(19) Europäisches Patentamt
European Patent Office
Office européen des brevets

(11) Veröffentlichungsnummer : **0 097 616**
**B1**

(12) # EUROPÄISCHE PATENTSCHRIFT

(45) Veröffentlichungstag der Patentschrift : **20.08.86**

(21) Anmeldenummer : **83810258.0**

(22) Anmeldetag : **13.06.83**

(51) Int. Cl.⁴ : **C 07 D211/46**, C 07 D211/94, C 07 D401/12, C 07 D211/72, C 07 D471/10, C 07 D491/113, C 07 D498/10, C 08 K 5/34, C 09 D 7/12

(54) **Polyalkylpiperidinsulfonsäureester.**

(30) Priorität : **17.06.82 CH 3743/82**

(43) Veröffentlichungstag der Anmeldung : **04.01.84 Patentblatt 84/01**

(45) Bekanntmachung des Hinweises auf die Patenterteilung : **20.08.86 Patentblatt 86/34**

(84) Benannte Vertragsstaaten : **CH DE FR GB IT LI**

(56) Entgegenhaltungen :
**EP-A- 0 006 213**
**EP-A- 0 008 102**
**EP-A- 0 028 318**
**EP-A- 0 055 216**
**DE-A- 2 717 087**
**US-A- 3 474 054**
**US-A- 3 503 982**

(73) Patentinhaber : **CIBA-GEIGY AG**
**Klybeckstrasse 141**
**CH-4002 Basel (CH)**

(72) Erfinder : **Slongo, Mario, Dr.**
**Sägetrainweg**
**CH-1712 Tafers (CH)**
Erfinder : **Karrer, Friedrich, Dr.**
**Rebbergstrasse 5**
**CH-4800 Zofingen (CH)**

**EP 0 097 616 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Polyalkylpiperidinsulfonsäureester und ihre Verwendung als Härtungskatalysatoren und Lichtschutzmittel für Lacksysteme auf Basis von säurehärtbaren Harzen.

Säurehärtbare Harze werden vor allem als Bindemittel für Lacke, Druckfarben und Anstrichfarben verwendet, wenn hohe Einbrenntemperaturen vermieden werden sollen. Säurehärtbare Harze können Aminoharze sein, einschliesslich verätherte, veresterte oder anderweitig modifizierte Melaminharze, Harnstoff-Formaldehydharze, Phenol-Formaldehydharze sowie Gemische solcher Harze mit Alkyd-, Polyester- oder Acrylharzen. Weitere säurehärtbare Harze sind Methylol-Verbindungen, Methyloläther von Polycarbonsäureimiden, z. B. Derivate von Polyacryl- oder Methacrylsäure, Urethanalkyde sowie Harze, die Carbonsäureester von N-Methylolimiden enthalten. Als saure Härtungskatalysatoren werden vorwiegend organische Säuren verwendet, darunter beispielsweise Sulfonsäuren, insbesondere p-Toluolsulfonsäure. Da diese Säuren bereits bei Raumtemperatur eine langsame Härtung bewirken, werden sie dem Harz erst kurz vor dessen Applikation zugesetzt, was mit den bekannten Problemen der Einhaltung bestimmter Topfzeiten verbunden ist. Zur Ermöglichung von Einkomponenten-Systemen hat man daher bereits die Verwendung maskierter Härtungskatalysatoren vorgeschlagen, aus denen bei erhöhter Temperatur die Säure freigesetzt wird. Beispiele hierfür sind Aminsalze von aromatischen Sulfonsäuren, wie die in der US-Patentschrift 3 474 054 vorgeschlagenen Pyridinsalze. Diese und andere Salze haben den Nachteil, dass sie bereits während der Lagerung eine langsame Härtung bewirken. Ausserdem entstehen dabei Geruchsprobleme.

Um die Lagerstabilität zu verbessern wurde in den DE-Offenlegungsschriften 2 345 114, 2 731 528 und 2 936 048 weiterhin vorgeschlagen, ein Reaktionsprodukt aus epoxidhaltigen Verbindungen mit organischen Sulfonsäuren als Härtungskatalysator zu verwenden.

Polyalkylpiperidinderivate sind allgemein als Lichtschutzmittel bekannt. So wurden solche Derivate in der EP-A1-6 213 als Lichtschutzmittel für sauerkatalysierte Einbrennlacke empfohlen. In der DE-OS 2 717 087 wurden auch schon Polyalkylpiperidinderivate beschrieben, die in der Seitenkette eine Sulfongruppe tragen können. Sulfonsäureester solcher Polyalkylpiperidine wurden jedoch bisher noch nicht beschrieben.

Es sind nun neue Polyalkylpiperidinsulfonsäureester gefunden worden, die zugleich als Härtungskatalysatoren und als Lichtschutzmittel eingesetzt werden können, bei denen die obenerwähnten Nachteile nicht auftreten. Ueberraschenderweise zerfallen diese Ester bei der gewünschten Härtungstemperatur unter Bildung der zur Härtung benötigten Sulfonsäure und des als Lichtschutzmittel im Lack verbleibenden Polyalkylpiperidinderivates.

Gegenstand der Erfindung sind demnach Verbindungen der Formel I und II

$$B—(X—O—SO_2—A)_m \qquad (I)$$

$$(B'—X—O—SO_2)_2—A' \qquad (II),$$

worin m die Zahlen 1 bis 3 bedeutet,

X eine direkte Bindung oder eine Gruppe der Formel III

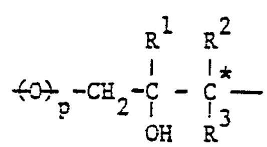

(III)

ist, die über C* an den Sauerstoff der Sulfogruppe gebunden ist, wobei, falls die Gruppe der Formel III an ein Stickstoffatom von B oder B' gebunden ist, p Null bedeutet und sonst p die Zahl 1 ist, und worin $R^1$, $R^2$ und $R^3$ unabhängig voneinander Wasserstoff oder Methyl bedeuten,

A unsubstituiertes oder durch Hydroxy substituiertes $C_1$-$C_{20}$ Alkyl, $C_1$-$C_4$ Alkoxy, $C_2$-$C_{12}$ Alkenyl, $C_7$-$C_{12}$ Aralkyl, $C_6$-$C_{10}$ Aryl, $C_7$-$C_{30}$ Alkaryl, $C_5$-$C_8$ Cycloalkyl, Trifluormethyl, Campheryl, eine Gruppe $—N(R^4)(R^5)$ oder $—C(CH_3)_2—CH_2—N(R^6)—C(O)—C(R^7) = CH_2$, worin $R^4$, $R^5$ und $R^6$ Wasserstoff oder $C_1$-$C_{12}$ Alkyl und $R^7$ Wasserstoff oder Methyl sind, eine Gruppe der Formel

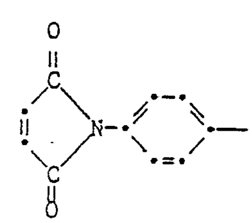

oder der Formel

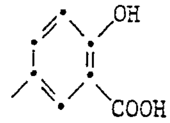

bedeuten,

A' $C_2$-$C_{20}$ Alkylen, $C_6$-$C_{12}$ Arylen oder $C_7$-$C_{28}$ Alkylarylen ist,

B bei m = 1 und B' eine Gruppe der Formeln IV bis X bedeuten,

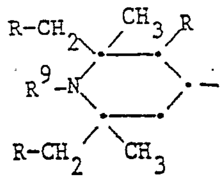

(IV)

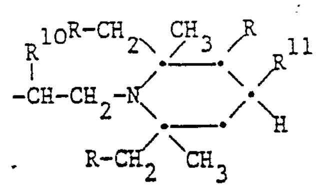

(V)

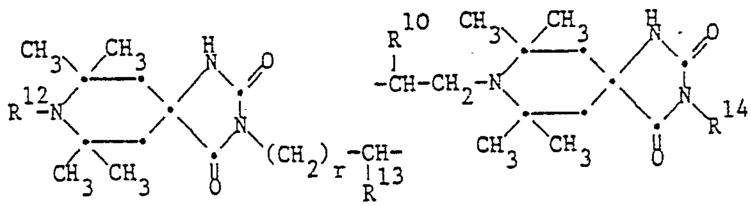

(VI)

(VII)

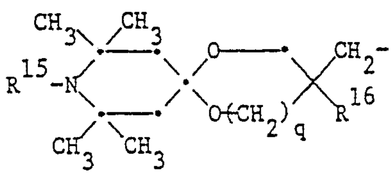

(VIII)

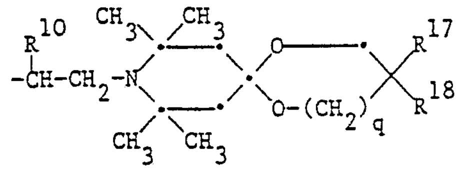

(IX)

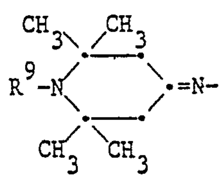

(X)

B bei m = 1 und B', falls X eine Gruppe der Formel III bedeutet, zusätzlich auch eine Gruppe der Formeln XI bis XVI sein kann,

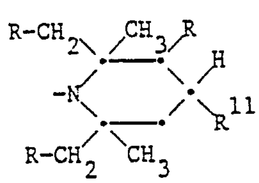

(XI)

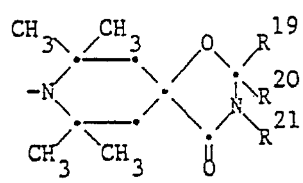

(XII)

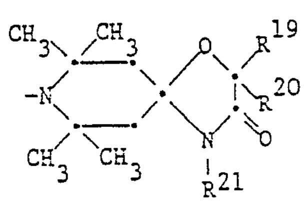

(XIII)

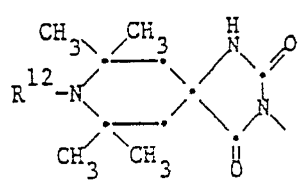

(XIV)

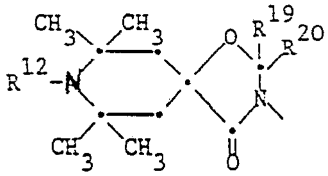

(XV)

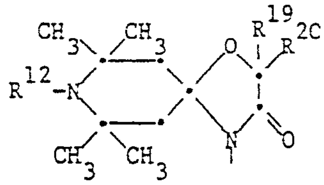

(XVI)

B bei m = 2 eine Gruppe der Formeln XVII bis XX bedeutet,

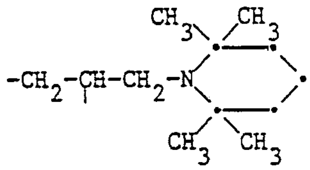

(XVII)

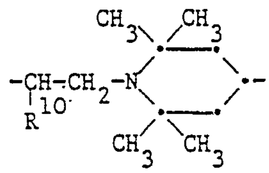

(XVIII)

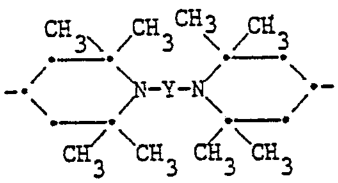

(XIX)

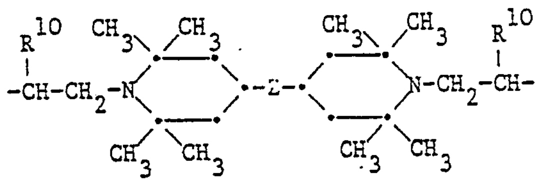

(XX)

B bei m = 2, falls X eine Gruppe der Formel III bedeutet, zusätzlich auch eine Gruppe der Formeln XXI bis XXV sein kann,

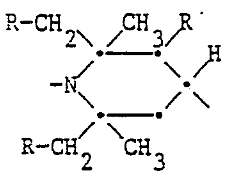

(XXI)

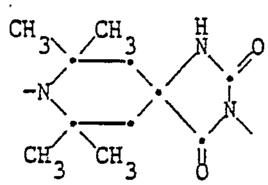

(XXII)

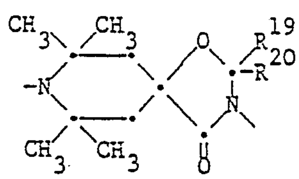

(XXIII)

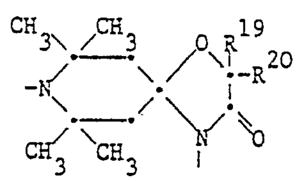

(XXIV)

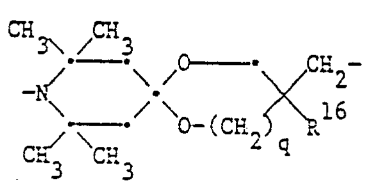

(XXV)

B bei m = 3, eine Gruppe der Formel XXVI

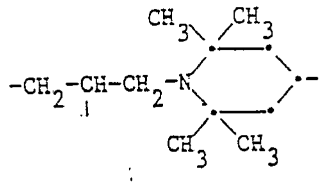

(XXVI)

bedeutet, wobei in den Formeln IV bis XXV

R Wasserstoff oder Methyl,

$R^9$ Wasserstoff, $C_1$-$C_{20}$ Alkyl, $C_3$-$C_5$ Alkenyl, $C_3$-$C_5$ Alkinyl, $C_7$-$C_{12}$ Aralkyl, $C_1$-$C_{18}$ Acyl, $C_3$-$C_{10}$ Alkoxycarbonylalkyl, $C_2$-$C_4$ Hydroxyalkyl, Cyanomethyl, Oxyl, eine Gruppe der Formel $—CH_2—CH(OH)—CH_2—OR^{27}$ oder eine Gruppe der Formel $—C(O)—N(R^{22})(R^{23})$,

$R^{10}$ Wasserstoff, $C_1$-$C_4$ Alkyl, $C_1$-$C_4$ Alkoxy, Phenyl, unsubstituiertes oder durch $C_1$-$C_4$ Alkyl im Phenylkern substituiertes Phenoxy, Phenoxymethyl,

$R^{11}$ Wasserstoff, Hydroxy, $C_1$-$C_{12}$ Alkoxy, $C_3$-$C_5$ Alkenyloxy, $C_1$-$C_{12}$ Acyloxy, $C_1$-$C_{12}$ Acylamino, $C_2$-$C_{14}$ Alkoxycarbonyl, eine Gruppe der Formel

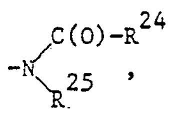 , $-C(O)-N(R^{24})(R^{25})$, $-OC(O)-N(R^{24})(R^{25})$,

oder

$$-CH_2-COOR^{24},$$

$R^{12}$ Wasserstoff, $C_1$-$C_{12}$ Alkyl, $C_3$-$C_5$ Alkenyl, $C_3$-$C_5$ Alkinyl, $C_7$-$C_{12}$ Aralkyl, $C_1$-$C_{18}$ Acyl, Cyanomethyl, $C_2$-$C_4$ Hydroxyalkyl,

$R^{13}$ Wasserstoff, $C_1$-$C_4$ Alkyl,

$R^{14}$ Wasserstoff, $C_1$-$C_{20}$ Alkyl, $C_3$-$C_5$ Alkenyl, $C_3$-$C_5$ Alkinyl, $C_7$-$C_{12}$ Aralkyl, $C_6$-$C_8$ Cycloalkyl,

$R^{15}$ Wasserstoff, $C_1$-$C_{12}$ Alkyl, $C_3$-$C_5$ Alkenyl, $C_7$-$C_{12}$ Aralkyl, $C_1$-$C_{18}$ Acyl, Cyanomethyl, $C_2$-$C_4$ Hydroxyalkyl,

$R^{16}$ Wasserstoff, Methyl, Ethyl,

$R^{17}$ und $R^{18}$ unabhängig voneinander Wasserstoff, $C_1$-$C_6$ Alkyl oder $R^{17}$ und $R^{18}$ zusammen eine der Gruppen der Formeln

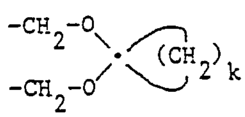 oder 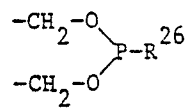 ,

$R^{19}$ und $R^{20}$ unabhängig voneinander Wasserstoff, $C_1$-$C_{12}$ Alkyl, unsubstituiertes oder durch $C_1$-$C_4$ Alkyl substituiertes Phenyl, $C_5$-$C_8$ Cycloalkyl, $C_7$-$C_{12}$ Aralkyl oder $R^{19}$ und $R^{20}$ zusammen $C_4$-$C_{11}$ Alkylen,

$R^{21}$ Wasserstoff, $C_1$-$C_{12}$ Alkyl, $C_3$-$C_5$ Alkenyl, $C_1$-$C_{18}$ Acyl, $C_7$-$C_{12}$ Aralkyl,

$R^{22}$ und $R^{23}$ unabhängig voneinander Wasserstoff, $C_1$-$C_{12}$ Alkyl, $C_3$-$C_5$ Alkenyl, Phenyl, Cyclohexyl,

$R^{24}$ $C_1$-$C_{12}$ Alkyl,

$R^{25}$ Wasserstoff oder $C_1$-$C_{12}$ Alkyl,

$R^{26}$ $C_1$-$C_4$ Alkyl, $C_1$-$C_{12}$ Alkoxy, Phenyl, Benzyl, Phenoxy,

$R^{27}$ $C_1$-$C_4$ Alkyl oder Phenyl,

r die Zahlen 1 bis 4,

q die Zahlen Null oder 1

k die Zahlen 4 bis 11,

Y Ethylen, 2-Butenylen-1,4, o-, p- oder m-Xylylen,

Z eine Gruppe der Formeln $—O—R^{28}—O—$, $—OC(O)—R^{29}—C(O)O—$, $—N(R^{30})—C(O)—R^{31}—C(O)—N(R^{30})—$, $—N(R^{30})—C(O)—C(O)—N(R^{30})—$,

$-C(O)O-R^{31}-OC(O)-$, 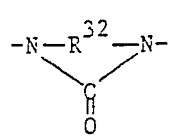

$R^{28}$ $C_2$-$C_{20}$ Alkylen, o-, m- oder p-Xylylen, 1,2-, 1,3- oder 1,4-Dimethylencyclohexylen,

$R^{29}$ $C_2$-$C_{20}$ Alkylen, o-, m- oder p-Phenylen, $C_8$-$C_{12}$ Arylendialkylen,

$R^{30}$ Wasserstoff, $C_1$-$C_{12}$ Alkyl, $C_7$-$C_{12}$ Aralkyl, $C_5$-$C_8$ Cycloalkyl, unsubstituiertes oder durch $C_1$-$C_4$ Alkyl substituiertes Phenyl,

$R^{31}$ $C_2$-$C_{20}$ Alkylen, unsubstituiertes oder durch $C_1$-$C_4$ Alkyl substituiertes o-, m- oder p-Phenylen, 1,2-, 1,3- oder 1,4-Cyclohexylen,

$R^{32}$ Ethylen oder Propylen bedeuten

sowie deren Säureadditionssalze.

Bedeuten etwaige Substituenten Alkyl, so handelt es sich um geradkettige oder verzweigte Alkylgruppen. Bedeuten sie $C_1$-$C_4$ Alkyl, dann handelt es sich z. B. um Methyl, Ethyl, n-Propyl, Isopropyl, n-Butyl, sec.-Butyl oder tert.-Butyl. Bedeuten sie $C_1$-$C_6$ Alkyl, so kommen zusätzlich z. B. n-Pentyl, 2,2-Dimethyl-propyl, n-Hexyl und 2,3-Dimethyl-butyl in Frage. Handelt es sich um $C_1$-$C_{12}$ Alkyl, so kann es sich ausserdem beispielsweise um n-Octyl, 1,1,3,3-Tetramethylbutyl, n-Nonyl, Decyl oder Dodecyl handeln. Bedeuten sie $C_1$-$C_{20}$ Alkyl, dann können sie überdies z. B. Tetradecyl, Hexadecyl, Heptadecyl, Octadecyl oder Eicosyl sein.

Bedeutet A $C_6$-$C_{10}$ Aryl, so kann es sich z. B. um Naphthyl und bevorzugt um Phenyl handeln.

Bedeuten etwaige Substituenten $C_3$-$C_5$ Alkenyl, so stellen sie beispielsweise Allyl, Methallyl, 2-Butenyl oder 2-Pentenyl dar. Bevorzugt ist Allyl. A und A′ bedeuten als $C_2$-$C_{12}$ Alkenyl zusätzlich z. B. Vinyl, 2-Hexenyl, 2-Octenyl oder 2-Dodecenyl. Bevorzugt ist Vinyl.

$R^9$, $R^{12}$ und $R^{14}$ bedeuten als $C_3$-$C_5$ Alkinyl z. B. Propargyl, 1-Butinyl, 2-Butinyl oder n-1-Pentinyl. Bevorzugt ist Propargyl.

Bedeuten etwaige Substituenten $C_5$-$C_8$ Cycloalkyl, so handelt es sich beispielsweise um Cyclopentyl, Cycloheptyl, Cyclooctyl und insbesondere um Cyclohexyl.

A und $R^{10}$ sind als $C_1$-$C_4$ Alkoxy z. B. Methoxy, Ethoxy, Isopropoxy, Butoxy. $R^{11}$ und $R^{26}$ können als $C_1$-$C_{12}$ Alkoxy beispielsweise zusätzlich auch Hexyloxy, Octyloxy oder Dodecyloxy bedeuten.

Bedeuten etwaige Substituenten $C_7$-$C_{12}$ Aralkyl, so handelt es sich z. B. um Phenalkyl oder Naphthalkyl, wobei der Alkylteil bei Phenalkyl jeweils aus verzweigtem oder unverzweigtem $C_1$-$C_6$ insbesondere jedoch $C_1$-$C_4$ Alkyl besteht. Bevorzugte Phenalkylreste sind $\alpha,\alpha$-Dimethylbenzyl, 1-Phenylethyl, 2-Phenylethyl und Benzyl. Beispiele für Naphthalkylreste sind 1-Naphthylmethyl, 1-Naphthylethyl, 2-Naphthylmethyl und 2-Naphthylethyl.

A ist als $C_7$-$C_{30}$ Alkaryl z. B. ein- bis dreimal durch Alkyl substituiertes Phenyl oder Naphthyl, wobei der Alkylteil jeweils aus verzweigtem oder unverzweigtem $C_1$-$C_{12}$ Alkyl besteht. Beispiele für Alkarylreste sind Tolyl, 2,4-Dimethylphenyl, 2,6-Dimethylphenyl, 2,4-Diethylphenyl, 2,6-Diethylphenyl, 2,4-Di-tert.butylphenyl, 2,6-Di-tert.-butylphenyl, 2,4,6-Triisopropylphenyl, 4-tert.-Butylphenyl. Bevorzugt sind p-Tolyl, p-Isopropylphenyl, p-Dodecylphenyl, 2,4,6-Trimethylphenyl, Di-nonylnaphthyl.

Bedeutet A Campheryl, so handelt es sich bevorzugt um Campher-10-yl.

$R^9$, $R^{12}$, $R^{15}$ und $R^{21}$ bedeuten als $C_1$-$C_{18}$ Acylrest z. B. den Rest von Essig-, Propion-, Butter-, Isovalerian-, Acryl-, Methacryl-, Capron-, Capryl-, Laurin-, Palmitin-, Oel-, Stearin-, Benzoe-, 4-Chlorbenzoe-, 4-Octylbenzoe-, Toluyl-, Phenoxyessig-, Salicyl-, 2-Phenylpropion-, Cyclohexancarbon-, Furan-2-carbon-, Dimethylcarbamin-, Diphenylcarbamin-, Cyclohexylcarbamin- oder Diphenylphosphinsäure. Bevorzugt sind Acetyl und Acryloyl.

A′, $R^{28}$, $R^{29}$ und $R^{30}$ bedeuten als $C_2$-$C_{20}$ Alkylen z. B. 1,2-Ethylen, 1,3-Propylen, Tetra-, Hexa-, Octa-, Dodeca- oder Octadecamethylen.

$R^{19}$ und $R^{20}$ können zusammen $C_4$-$C_{11}$ Alkylen sein. Sie bilden dann zusammen mit dem C-Atom, an dem sie gebunden sind, einen Cycloalkanring, z. B. Cyclopentan-, Cyclohexan-, Cyclooctan- oder Cyclododecanring.

A′ bedeutet als $C_6$-$C_{12}$ Arylen z. B. Phenylen, Naphthylen oder Diphenylen. Bevorzugt ist m-Phenylen.

A′ als $C_7$-$C_{28}$ Alkylarylen kann z. B. Methylphenylen, Dimethylphenylen oder Dinonylnapthylen sein.

Als $C_2$-$C_4$ Hydroxyalkyl können $R^9$, $R^{12}$ une $R^{15}$ z. B. 2-Hydroxyethyl, 3-Hydroxypropyl oder 4-Hydroxybutyl sein. Bevorzugt ist 2-Hydroxyethyl.

$R^9$ ist als $C_3$-$C_{10}$ Alkoxycarbonylalkyl z. B. Methoxycarbonylmethyl, Ethoxycarbonylmethyl, Isopropoxycarbonylmethyl, Octyloxycarbonylmethyl, Methoxycarbonylethyl, Ethoxycarbonylethyl oder Butoxycarbonylethyl.

$R^{11}$ bedeutet als $C_3$-$C_5$ Alkenyloxy z. B. Allyloxy, Methallyloxy, 2-Butenyloxy oder 2-Pentenyloxy.

$R^{11}$ bedeutet als $C_1$-$C_{12}$ Acyloxy beispielsweise Formyloxy, Acetyloxy, Propionyloxy, Butyroyloxy, Hexanoyloxy, Octanoyloxy oder Lauroyloxy.

Als $C_1$-$C_{12}$ Acylamino kann $R^{11}$ z. B. Formylamino, Acetylamino, Propionylamino, Butyroylamino, Hexanoylamino, Octanoylamino oder Lauroylamino bedeuten.

Als $C_2$-$C_{14}$ Alkoxycarbonyl bedeutet $R^{11}$ z. B. Methoxycarbonyl, Ethoxycarbonyl, Butoxycarbonyl, Dodecyloxycarbonyl.

$R^{29}$ kann als $C_8$-$C_{12}$ Arylendialkylen z. B. Xylylen oder Phenylen-1,4-diethylen sein.

Von besonderem Interesse sind Verbindungen der Formel I, worin m die Zahlen 1 oder 2 bedeutet,

X eine direkte Bindung oder eine Gruppe der Formel III, worin $R^1$, $R^2$ und $R^3$ Wasserstoff sind, bedeutet,

A $C_1$-$C_{18}$ Alkyl, Methoxy, Vinyl, Amino, Phenyl, Naphthyl, 1 oder 2 mal durch $C_1$-$C_{12}$ Alkyl substituiertes Phenyl oder Naphthyl, 2,4,6-Trimethylphenyl, Campher-10-yl, eine Gruppe der Formel $-C(CH_3)_2-CH_2-NH-C(O)-CH=CH_2$ oder eine Gruppe der Formel

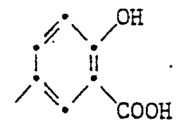

ist,

B bei m = 1 eine Gruppe der Formeln IV und V ist, bei m = 1, falls X eine Gruppe der Formel III bedeutet, zusätzlich auch eine Gruppe der Formeln XI und XIV ist,

bei m = 2 eine Gruppe der Formeln XVIII und XX ist und

bei m = 2, falls X eine Gruppe der Formel III bedeutet, zusätzlich auch eine Gruppe der Formel XXI bedeutet,

wobei in den Formeln IV, V, XI, XIV, XVIII, XX und XXI

R Wasserstoff,

$R^9$ Wasserstoff, $C_1$-$C_6$ Alkyl, Allyl, Benzyl, Acetyl, Acryloyl, 2-Hydroxyethyl, Cyanomethyl oder Oxyl,

$R^{10}$ Wasserstoff, Methyl, Phenyl, Phenoxymethyl,

$R^{11}$ Wasserstoff, $C_1$-$C_4$ Alkoxy, Allyloxy, $C_1$-$C_{12}$ Acyloxy, $C_1$-$C_{12}$ Acylamino, $C_2$-$C_6$ Alkoxycarbonyl oder eine Gruppe der Formel

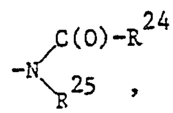
,

$R^{12}$ Wasserstoff oder $C_1$-$C_4$ Alkyl,

$R^{13}$ Wasserstoff,

$R^{14}$ Wasserstoff, $C_1$-$C_{12}$ Alkyl, Allyl oder Benzyl,

$R^{15}$ Wasserstoff, Methyl oder Benzyl,

$R^{16}$ Wasserstoff, Methyl oder Ethyl,

$R^{17}$ und $R^{18}$ $C_1$-$C_4$ Alkyl,

$R^{19}$ und $R^{20}$ Wasserstoff, $C_1$-$C_6$ Alkyl oder zusammen $C_4$-$C_{11}$ Alkylen,

$R^{24}$ $C_1$-$C_{12}$ Alkyl

$R^{25}$ Wasserstoff oder $C_1$-$C_{12}$ Alkyl sind,

Z eine Gruppe der Formeln $-OC(O)-R^{29}-C(O)O-$ oder $-N(R^{30})-C(O)-R^{31}-C(O)-N(R^{30})-$ bedeutet,

worin

$R^{29}$ $C_2$-$C_{10}$ Alkylen, o-, m- oder p-Phenylen,

$R^{30}$ Wasserstoff, $C_1$-$C_{12}$ Alkyl oder Benzyl und

$R^{31}$ $C_2$-$C_{10}$ Alkylen, o-, m- oder p-Phenylen sind,

sowie deren Säureadditionssalze.

Von ganz besonderem Interesse sind Verbindungen der Formel I, worin m die Zahlen 1 oder 2 bedeutet,

X eine direkte Bindung oder eine Gruppe der Formel III, worin $R^1$, $R^2$ und $R^3$ Wasserstoff sind, bedeutet,

A Methyl, Ethyl, $C_{14}$-$C_{17}$ Alkyl, Methoxy, Vinyl, Amino, Phenyl, p-Methylphenyl, p-Isopropylphenyl, p-Dodecylphenyl, Naphthyl, Dinonylnaphthyl, Campher-10-yl, eine Gruppe der Formel $-C(CH_3)_2-CH_2-NH-C(O)-CH=CH_2$ oder eine Gruppe der Formel

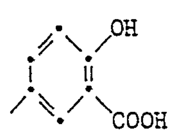

ist,

B bei m = 1 eine Gruppe der Formeln IV und V,

bei m = 1, falls X eine Gruppe der Formel III bedeutet, zusätzlich auch eine Gruppe der Formel XI,

bei m = 2 eine Gruppe der Formel XVIII,

bei m = 2, falls X eine Gruppe der Formel III bedeutet, zusätzlich auch eine Gruppe der Formel XXI bedeutet,

wobei in den Formeln IV, V, XI, XVIII und XXI

R Wasserstoff,

$R^9$ Wasserstoff, $C_1$-$C_6$ Alkyl, Allyl, Benzyl, Oxyl, Acetyl, Acryloyl, β-Hydroxyethyl oder Cyanomethyl,
$R^{10}$ Wasserstoff, Methyl oder Phenyl und
$R^{11}$ Wasserstoff oder $C_1$-$C_4$ Alkoxy sind,
sowie deren Säureadditionssalze.

Bevorzugt sind darunter Verbindungen der Formel I, worin m die Zahl 1 bedeutet, B eine Gruppe der Formel IV ist, X eine direkte Bindung oder eine Gruppe der Formel $—O—CH_2—CH(OH)-CH_2—$ ist und A die oben gegebene Bedeutung hat.

Einige Beispiele von erfindungsgemässen Verbindungen der Formeln I und II sind in folgenden Tabellen 1 und 2 aufgeführt.

Tabelle 1

$B \leftarrow (X - OSO_2 - A)_m$

| B | X | A | m |
|---|---|---|---|
| 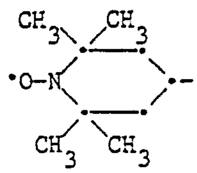 | direkte Bindung | 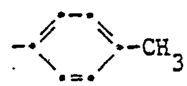 | 1 |
| 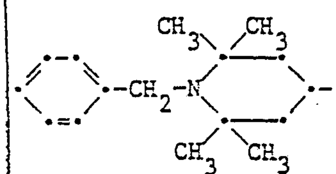 | direkte Bindung | 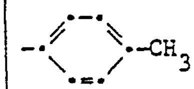 | 1 |
| 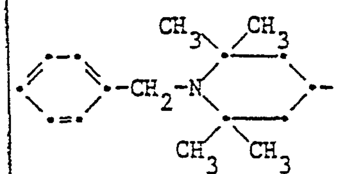 | direkte Bindung | $-CH_3$ | 1 |
| 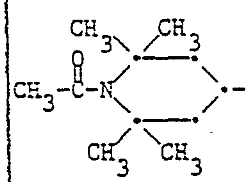 | direkte Bindung | 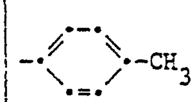 | 1 |
| 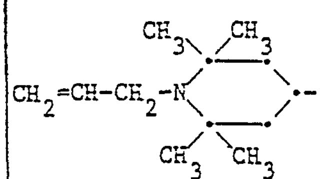 | direkte Bindung | 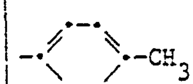 | 1 |
| 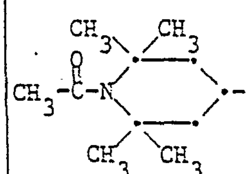 | $-O-CH_2-\underset{OH}{CH}-CH_2-$ | 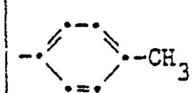 | 1 |

Tabelle 1 (Fortsetzung)

$B-(X - OSO_2 - A)_m$

| B | X | A | m |
|---|---|---|---|
| 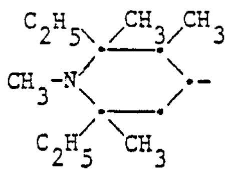 | direkte Bindung | $-OCH_3$ | 1 |
| 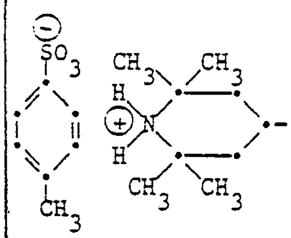 | direkte Bindung | 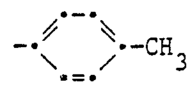 | 1 |
| 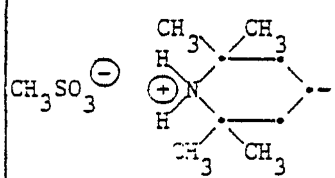 | direkte Bindung | $-CH_3$ | 1 |
| 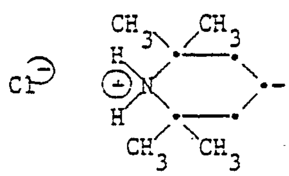 | direkte Bindung | 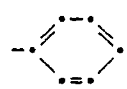 | 1 |
| 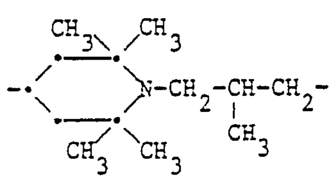 | direkte Bindung | $-CH_3$ | 2 |
| 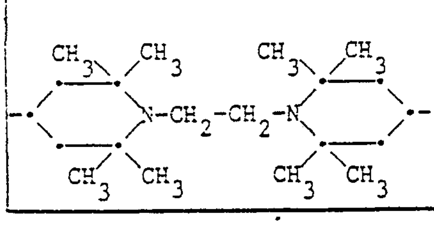 | direkte Bindung | 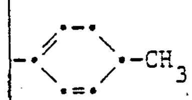 | 2 |

Tabelle 1 (Fortsetzung)

| $B-(X-OSO_2-A)_m$ | | | |
|---|---|---|---|
| B | X | A | m |
| 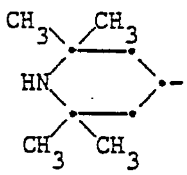 | direkte Bindung | Campher-10-yl | 1 |
| 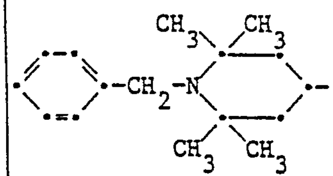 | $-O-CH_2-\underset{OH}{CH}-CH_2-$ | 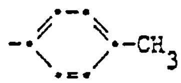 | 1 |
| 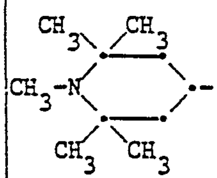 | $-O-CH_2-\underset{OH}{CH}-CH_2-$ | 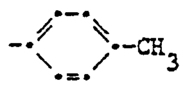 | 1 |
| 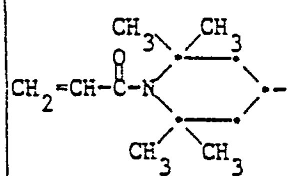 | direkte Bindung | $-C_2H_5$ | 1 |
| 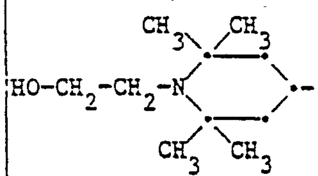 | direkte Bindung | 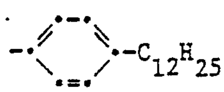 | 1 |
| 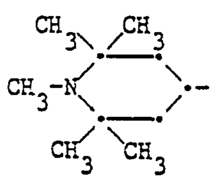 | direkte Bindung | $-NH_2$ | 1 |
| 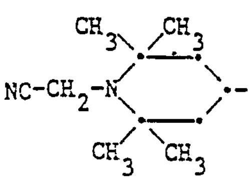 | direkte Bindung | 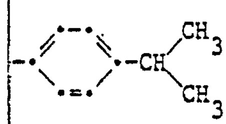 | 1 |

Tabelle 1 (Fortsetzung)

$B{-}(X - OSO_2 - A)_m$

| B | X | A | m |
|---|---|---|---|
| 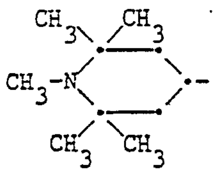 | direkte Bindung | 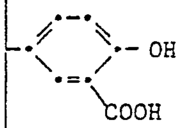 | 1 |
| 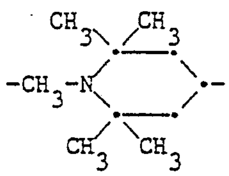 | direkte Bindung | $-CH=CH_2$ | 1 |
| 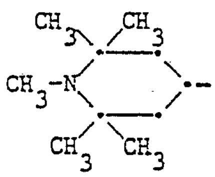 | direkte Bindung | $-C_{14-17}H_{29-35}$ | 1 |
| 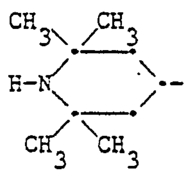 | direkte Bindung | $-C(CH_3)_2-CH_2-NH-C(O)-CH=CH_2$ | 1 |
| 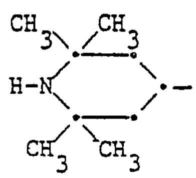 | direkte Bindung | 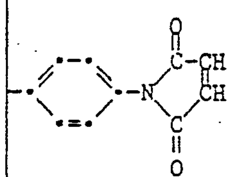 | 1 |

Tabelle 2

$(B' - X - OSO_2 -)_2\ A'$

| B' | X | A' |
|---|---|---|
| 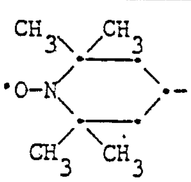 | direkte Bindung | 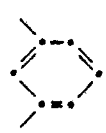 |
| 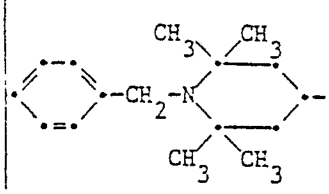 | direkte Bindung | $-CH_2-CH_2-$ |

Tabelle 2 (Fortsetzung)

$(B' - X - OSO_2 \rightarrow_2 A'$

| B' | X | A' |
|---|---|---|
| 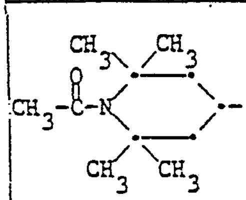 | direkte Bindung | $-(CH_2)_6-$ |
| 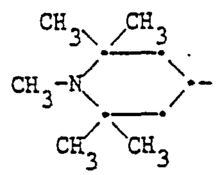 | direkte Bindung | 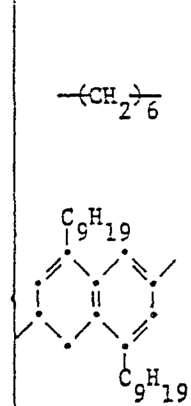 |
| 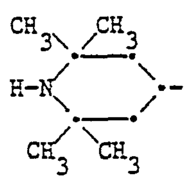 | $-O-CH_2-CH(OH)-CH_2-$ | $-CH_2-CH_2-$ |

Die Herstellung der erfindungsgemässen Verbindungen der Formeln I und II erfolgt in Analogie zu an sich bekannten Verfahren z. B.

a) dadurch, dass man einen Alkohol der Formel

B—OH (XXVII)

mit einem Sulfonsäurehalogenid, insbesondere Chlorid, der Formel

$A—SO_2Cl$ (XXVIII)

im Molverhältnis 1 : 1 oder

einen Alkohol der Formel

B'—OH (XXIX)

mit einem Disulfonsäurehalogenid, insbesondere Chlorid, der Formel

$Cl—O_2S—A—SO_2—Cl$

im Molverhältnis 2 : 1

in einem inerten Lösungsmittel in Gegenwart einer Base als Protonenakzeptor bei einer Temperatur zwischen 0 und 100 °C, bevorzugt zwischen 20 und 50 °C, umsetzt, wobei in den Formeln XXVII-XXIX A, B und B' die oben angegebene Bedeutung haben.

Als Basen kommen in Frage tertiäre Amine wie Trialkylamine, Pyridin, Alkalihydroxide oder -carbonate und Erdalkalihydroxide. Es kann aber auch ein entsprechender Ueberschuss (1 oder 2 Mol) der zu veresternden Polyalkylpiperidinbase eingesetzt werden, wobei die eine Hälfte verestert wird und die andere als Protonenakzeptor wirkt.

b) dadurch, dass man einen Alkohol der Formel XXVII mit einem Sulfonsäureanhydrid der Formel

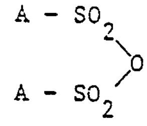 (XXX)

in einem inerten Lösungsmittel unter Bildung von Verbindungen der Formel I und der Formel A—$SO_2OH$ umsetzt, wobei A und B die oben angegebene Bedeutung haben.

c) dadurch, dass man einen Alkohol der Formel XXVII mit einem Sulfonsäureester der Formel

$$A—SO_2OR^{33} \quad (XXXI)$$

im Molverhältnis 1 : 1 oder
einen Alkohol der Formel XXIX mit einem Disulfonsäureester der Formel

$$R^{33}OSO_2—A'—SO_2OR^{33} \quad (XXXII)$$

im Molverhältnis 2 : 1 bzw.
ein Diol der Formel

$$HO—B—OH \quad (XXXIII)$$

mit einem Sulfonsäureester der Formel XXXI im Molverhältnis 1 : 2 in Gegenwart einer Titanverbindung der Formel $Ti(OR^{34})_4$ umsetzt, wobei A, A' B und B' die oben angegebene Bedeutung haben und $R^{33}$ und $R^{34}$ $C_1$-$C_4$-Alkyl sind.

d) dadurch, dass man ein Monoepoxid der Formel

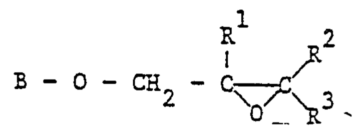

(XXXIV)

mit einer Sulfonsäure der Formel

$$A—SO_2—OH \quad (XXXV)$$

im Molverhältnis 1 : 1 oder
ein Monoepoxid der Formel

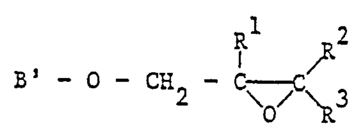

(XXXVI)

mit einer Disulfonsäure der Formel

$$HO—O_2S—A'—SO_2—OH \quad (XXXVII)$$

im Molverhältnis 2 : 1 bzw.
ein Diepoxid der Formel

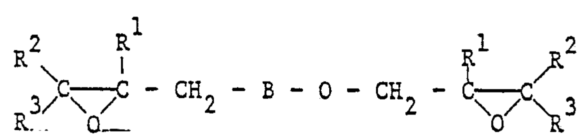

(XXXVIII)

mit einer Sulfonsäure der Formel XXXV im Molverhältnis 1 : 2 in einem inerten Lösungsmittel umsetzt.

Die Ausgangsprodukte sind Bekannt. Sollten einige von ihnen neu sein, so können sie in Analogie zu an sich bekannten Methoden hergestellt werden und stellen auch einen Gegenstand der Erfindung dar.

Säureadditionssalze erhält man durch Umsetzung der erfindungsgemässen Polyalkylpiperidinsulfonsäureester, die eine basische Polyalkylpiperidingruppe enthalten mit Säuren. Geeignete Säuren sind z. B. anorganische Säuren wie beispielsweise Salzsäure, Phosphorsäure, Amidosulfonsäure oder insbesondere organische Säuren wie z. B. Mono- oder Polysulfonsäuren, beispielsweise Methansulfonsäure, Methylamidosulfonsäure, Ethansulfonsäure, 2-Ethylhexansulfonsäure, Vinylsulfonsäure, Toluol- oder Naphthalinsulfonsaüren, Benzol- oder Naphthalin-disulfonsäuren, Styrolsulfonsäure, alkylierte Benzol- oder Naphthalinsulfonsäuren, technische Gemische von Paraffinsulfonsäuren, Acrylamido-N-alkansulfonsäuren, Polystyrolpolysulfonsäuren oder Polyvinylsulfonsäure ; saure Phosphorsäureester wie z. B. Phenylphosphorsäure oder Diethylphosphorsäure ; Phosphon- und Phosphinsäuren wie z. B. Methylphosphonsäure, Phenylphosphonsäure, Dodecylphenylphosphonsäure, Phenyl-butyl-phosphinsäure, Diphenylphosphinsäure oder Methyl-benzyl-phosphinsäure ; Phosphonsäuremonoester wie z. B.

Methyl-methylphosphonat oder Ethylphenylphosphonat ; Maleinsäure und deren Monoester wie z. B. Monobutylmaleat oder Monohexylmaleat ; und Phthalsäure und deren Monoester wie z. B. Monohexylphthalat.

Ein weiterer Gegenstand der Erfindung ist die Verwendung der Verbindungen der Formeln I und II als Härtungskatalysatoren von säurehärtbaren Harzen, wobei sie in diesen Harzen zugleich als Lichtschutzmittel dienen, sowie die die erfindungsgemässen Verbindungen enthaltenden säurehärtbaren Harze.

Die Polyalkylpiperidinsulfonsäureester der Formeln I und II werden den Harzen in einer für die Härtung ausreichenden Menge zugesetzt. Die benötigte Menge hängt nicht nur von der Art des Harzes sondern auch von der beabsichtigten Härtungstemperatur und Härtungszeit ab. Im allgemeinen verwendet man 0,1 bis 10 Gew.%, vorzugsweise 0,5 bis 5 Gew.%, bezogen auf das lösungsmittelfreie Harz. In diesen Mengen ist auch eine ausreichende Lichtschutzwirkung der Piperidinsulfonsäureester gewährleistet. Mischungen von Verbindungen der Formeln I und II können auch eingesetzt werden. Die Zugabe der Piperidinsulfonsäureester zum Lack geschieht durch einfaches Mischen mit den übrigen Komponenten des Lackes. Mann kann den Ester auch in gelöster Form dem Lack zugeben. Dies ist besonders dann von Vorteil, wenn bei Herstellung des Esters dieses als Lösung anfällt, so dass seine Isolierung nicht notwendig ist.

Als säurehärtbare Harze kommen alle Harze in Frage, deren Härtung durch saure Katalysatoren beschleunigt werden kann. Das sind vor allem Lacke auf Basis von Acryl-, Polyester-, Alkyd-, Melamin-, Harnstoff-, und Phenolharzen, insbesondere aber die Mischungen von Acryl-, Polyester- oder Alkydharzen untereinander oder mit einem Melaminharz. Darunter fallen auch modifizierte Lackharze wie z. B. acrylmodifizierte Polyester- oder Alkydharze. Beispiele für einzelne Typen von Harzen, die unter den Begriff Acryl-, Polyester- und Alkydharze fallen, sind z. B. in Wagner, Sarx/Lackkunstharze (München, 1971), Seiten 86 bis 123 und 229 bis 238, oder in Ullmann/Encyclopädie der techn. Chemie, 4. Auflage, Band 15 (1978), Seiten 613 bis 628, beschrieben. Von besonderer Bedeutung ist die saure Katalyse für die Härtung von Lacken, die verätherte Aminoharze enthalten, wie z. B. methylierte oder butylierte Melaminharze (N-Methoxymethyl- bzw. N-Butoxymethylmelamin) oder methylierte/butylierte Glycolurile usw.

z. B. :

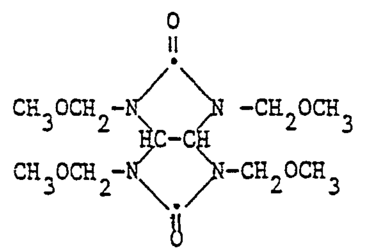

Weitere Harzzusammensetzungen sind Gemische von polyfunktionellen Alkoholen, oder Hydroxylgruppen enthaltenden Acryl- und Polyesterharzen, oder partiell verseiftem Polyvinylacetat oder Polyvinylalkohol mit polyfunktionellen Dihydropyranyläthern, wie beispielsweise Derivate der 3,4-Dihydro-2H-pyran-2-carbonsäure. Besonders bevorzugt für den Einsatz der erfindungsgemässen Piperidinsulfonsäureester sind Harze, die eine Acrylatkomponente enthalten und mit vollverätherten Melaminharzen gehärtet werden.

Für bestimmte Zwecke verwendet man auch Harzzusammensetzungen, die monomere oder oligomere Bestandteile mit polymerisationsfähigen ungesättigten Gruppen haben. Auch solche Harzzusammensetzungen sind mit den erfindungsgemässen Verbindungen härtbar. Hierbei können zusätzlich radikalische Polymerisationsinitiatoren oder Photoinitiatoren, beispielsweise aus der Klasse der aromatischen Ketone, Benzoinverbindungen, Benzilketale oder α-Hydroxyacetophenonderivate, mitverwendet werden. Zusätzlich zur Polymerisation der ungesättigten Komponenten muss aber stets eine sauer katalysierte Vernetzung (eventuell beim Einbrennen) erfolgen.

Die Lacke können Lösungen oder Dispersionen des Lackharzes in einem organischen Lösungsmittel oder in Wasser sein, sie können aber auch lösungsmittelfrei sein. Von besonderem Interesse sind Lacke mit geringem Lösungsmittelanteil, sogenannte « high solids-Lacke ». Die Lacke können Klarlacke sein, wie sie z. B. in der Automobilindustrie als Decklacke von Mehrschichten-Anstrichen verwendet werden. Sie können auch Pigmente enthalten, seien es anorganische oder organische Pigmente, sowie Metallpulver für Metalleffekt-Lacke.

Die Lacke können weiterhin kleinere Mengen an speziellen Zusätzen enthalten, wie sie in der Lacktechnologie üblich sind, beispielsweise Verlaufshilfsmittel, Thixotropiemittel oder Antioxydantien. Obwohl die erfindungsgemässen Piperidinsulfonsäureester hervorragende Lichtschutzmittel sind, kann es in bestimmten Fällen von Vorteil sein, zusätzlich noch andere Lichtschutzmittel mitzuverwenden, wie z. B. solche vom Typ der UV-Absorber oder der organischen Nickelverbindungen, weil dabei synergistische Effekte auftreten können. Beispiele für verwendbare Klassen von UV-Absorbern sind die Hydroxyphenylbenztriazole, die Hydroxybenzophenone, die Oxalanilide, die Cyanoacrylate und die Hydroxyphenyl-s-triazine. Beispiele für organische Nickelverbindungen sind Nickelsalze von Phosphorsäuremonoestern oder Ni-Komplexe von Bis-phenolen. Besonders bevorzugt ist die Mitverwendung von Hydroxyphenyl-benztriazol-Lichtschutzmitteln, wie z. B. von 2-(2-Hydroxy-3,5-di-t-amylphenyl)-benztriazol oder 2-[2-Hydroxy-3.5-di-(α-dimethylbenzyl)-phenyl]-benztriazol.

Die Lacke werden auf die zu beschichtenden Substrate nach den üblichen Methoden der industriellen Lackierung aufgetragen, beispielsweise durch Streichen, Besprühen oder Tauchen.

Die Lacke werden nach dem Auftragen getrocknet und eingebrannt. Die Härtungstemperaturen können bei 80° bis 300 °C liegen bei Härtungszeiten von einigen Sekunden bis zu einer Stunde. Für die Verwendung der erfindungsgemässen Verbindungen sind vor allem niedrige Härtungstemperaturen von etwa 100° bis 150 °C von Interesse, wie sie bei Ausbesserungslackierungen an Automobilen oder bei sonstigen Lackierungen empfindlicher Industrieartikel angewendet werden.

Die Verwendung der erfindungsgemässe Verbindungen enthaltenden Lacke ist für alle Arten der industriellen Lackierung geeignet, wie z. B. für die Lackierung von Maschinen, Fahrzeugen, Schiffen oder Konstruktionsteilen. Von besonderer Bedeutung ist sie für die Fahrzeug-Lackierung. Hierbei kann es sich sowohl um Einschicht-Lackierung wie Mehrschicht-Lackierung handeln. Von besonderem Interesse ist auch die Anwendung der erfindungsgemässe Verbindungen enthaltenden Lacke für die kontinuierliche Beschichtung von Blechen, beispielsweise Stahl- oder Aluminiumblechen, dem sogenannten Coil-Coat-Verfahren.

Im folgenden wird die Erfindung anhand einiger typischer Beispiele näher beschrieben.

### Beispiel 1

Zu einer Lösung von 24,74 g (0,1 Mol) 1-Benzyl-4-hydroxy-2,2,6,6-tetramethylpiperidin und 20,2 g (0,2 Mol) Triethylamin in 60 ml Toluol tropft man unter Rühren bei Raumtemperatur innerhalb einer halben Stunde 11,7 g (0,102 Mol) Methansulfonsäurechlorid in 50 ml Toluol, wobei die Temperatur bis auf ca. 70 °C ansteigt. Anschliessend wird 20 h bei Raumtemperatur nachgerührt. Das Reaktionsgemisch wird am Rotationsverdampfer im Wasserstrahlvakuum bei 40 °C eingedampft, der Rückstand in Dichlormethan gelöst, wiederholt mit Wasser gewaschen, die organische Phase über Natriumsulfat getrocknet und eingedampft. Die rohe Verbindung wird in Cyclohexan umkristallisiert, wodurch reines 4-Methansulfonyloxy-1-benzyl-2,2,6,6-tetramethylpiperidin vom Smp. 104-106 °C als farbloses Kristallpulver erhalten wird (Verbindung Nr. 1).

Analog werden die folgenden Verbindunge hergestellt :

1-Benzyl-4-p-toluolsulfonyloxy-2,2,6,6-tetramethylpiperidin, Smp. 103-104 °C (Verbindung Nr. 2).
1-Allyl-4-p-toluolsulfonyloxy-2,2,6,6-tetramethylpiperidin, Smp. 82-83 °C (Verbindung Nr. 3).
1-Acetyl-4-p-toluolsulfonyloxy-2,2,6,6-tetramethylpiperidin, Smp. 104-105 °C (Verbindung Nr. 4).
4-p-Toluolsulfonyloxy-1,2,2,6,6-pentamethylpiperidin, Smp. 73 °C (Verbindung Nr. 5).

### Beispiel 2

Eine Lösung von 17,2 g (0,1 Mol) 4-Hydroxy-2,2,6,6-tetramethylpiperidin-1-oxyl in 130 ml Pyridin wird bei ca. 0 °C unter Rühren portionenweise mit 19,1 g (0,1 Mol) p-Toluolsulfonsäurechlorid versetzt. Hierauf wird 24 h bei ca. 20 °C weiter gerührt. Zur Isolierung des Sulfonsäureesters wird das Reaktionsgemisch am Rotationsverdampfer im Vakuum bei 45 °C weitgehend vom Pyridin befreit, der Rückstand in Dichlormethan gelöst und wiederholt mit Wasser extrahiert. Die organische Phase trocknet man über Natriumsulfat und destilliert das Lösungsmittel ab. Der Rückstand wird bei 40-50 °C während einer Stunde kräftig mit Hexan verrührt, die Hexanlösung abfiltriert, eingedampft und der Rückstand in Diisopropylether umkristallisiert, wodurch das 4-p-Toluolsulfonyloxy-2,2,6,6-tetramethylpiperidin-1-oxyl vom Schmelzpunkt 112-114 °C erhalten wird (Verbindung Nr. 6).

Analog werden hergestellt :

4-Methansulfonyloxy-1,2,2,6,6-pentamethylpiperidin, Sdp. 80 °C/0, 13 Pa (Verbindung Nr. 7).
1-[2-(p-Toluolsulfonyloxy)ethyl]-2,2,6,6-tetramethylpiperidin, Smp. 106-108 °C (Verbindung Nr. 8).

### Beispiel 3

15,2 g 4-(2,3-Epoxypropyloxy)-1-benzyl-2,2,6,6-tetramethylpiperidin werden mit 9,6 g p-Toluolsulfonsäure in 50 ml Toluol zusammengegeben. Unter Stickstoff wird auf 80 °C erwärmt. Man lässt 2 h bei dieser Temperatur reagieren. Das Dünnschichtchromatogramm zeigt kein Ausgangsprodukt mehr. Man kühlt nun die hellgelbe Lösung ab. Es entstehen zwei flüssige Schichten. Unter Zugabe von ca. 100 ml. Methylenchlorid erhält man eine homogene hellgelbe Lösung, die über $MgSO_4$ sicc getrocknet wird. Die organische Lösung wird darauf vollständig eingedampft und am Hochvakuum bei 40-45 °C getrocknet. Man erhält 23,5 g p-Toluolsulfonsäure-[2-hydroxy-3-(1-benzyl-2,2,6,6-tetramethyl-4-piperidyloxy)propyl]-ester als leicht oranges Harz (Verbindung Nr. 9).

| | | | |
|---|---|---|---|
| Analyse : | Ber. : | C 65,66 % | Gef. : C 65,48 % |
| | | H 7,84 % | H 8,20 % |

| | | | |
|---|---|---|---|
| N | 2,94 % | N | 2,83 % |
| S | 6,74 % | S | 6,38 % |

Analog werden die folgenden Sulfonsäureester aus den entsprechenden Glycidylethern hergestellt :

p-Toluolsulfonsäure-[2-hydroxy-3-(1,2,2,6,6-pentamethyl-4-piperidyloxy)-propyl]-ester, amorphers Harz (Verbindung Nr. 10).

p-Dodecylbenzolsulfonsäure-[2-hydroxy-3-(1,2,2,6,6-pentamethyl-4-piperidyloxy)-propyl]-ester, amorphes Harz (Verbindung Nr. 11).

p-Dodecylbenzolsulfonsäure-[2-hydroxy-3-(1-benzyl-2,2,6,6-tetramethyl-4-piperidyloxy)-propyl]-ester, amorphes Harz (Verbindung Nr. 12).

p-Toluolsulfonsäure-[2-hydroxy-3-(1-acetyl-2,2,6,6-tetramethyl-4-piperidyloxy)-propyl]-ester, viskoses Oel (Verbindung Nr. 13).

p-Dodecylbenzolsulfonsäure-[2-hydroxy-3-(4-hydroxy-2,2,6,6-tetramethylpiperidino)-propyl]-ester, amorphes Harz (Verbindung Nr. 14).

## Beispiel 4

Herstellung der Glycidylether 30,3 g (0,3 Mol) 4-Hydroxy-1-benzyl-2,2,6,6-tetramethylpiperidin werden in 250 ml Toluol gelöst. Dazu gibt man 84 g (1,5 Mol) festes, pulverisiertes KOH und 5 Mol% 18-Crown-6. Man erwärmt auf 70 °C und gibt bei dieser Temperatur langsam eine Lösung von 50 g (0,5 Mol) Epichlorhydrin in 50 ml Toluol gelöst hinzu. Die Reaktion ist sehr exotherm. Die Innentemperatur soll 75-80 °C nicht übersteigen. Die Reaktionslösung wird mit der Zeit bräunlich gefärbt. Man verfolgt den Umsatz mittels Gaschromatographie. Nach dem Zutropfen während ca 1 h lässt man noch 2 h bei 75-80 °C ausrühren. Man kühlt danach die Reaktionslösung ab und versetzt sie mit Wasser. Die Toluolphase wird abgetrennt und eingedampft. Der Rückstand wird am Hochvakuum destilliert. Man erhält 50,1 g 4-(2,3-Epoxypropyloxy)-1-benzyl-2,2,6,6-tetramethylpiperidin. Sdp. 137-140 °C/6,66 Pa.

Verwendet man anstelle von 4-Hydroxy-1-benzyl-2-2,6,6-tetramethylpiperidin die folgenden Piperidine und verfährt im übrigen wie oben beschrieben, so erhält man

aus 4-Hydroxy-1,2,2,6,6-pentamethylpiperidin 4-(2,3-Epoxypropyloxy)-1,2,2,6,6-pentamethylpiperidin, Sdp. 68-69 °C/0,666 Pa

aus 4-Hydroxy-1-allyl-2,2,6,6-tetramethylpiperidin 4-(2,3-Epoxypropyloxy)-1-allyl-2,2,6,6-tetramethylpiperidin, Sdp. 88-90 °C/1, 333 Pa

aus 4-Hydroxy-1-acetyl-2,2,6,6,-tetramethylpiperidin 4-(2,3-Epoxypropyloxy)-1-acetyl-2,2,6,6-tetramethylpiperidin Sdp. 110 °C/13,332 Pa

aus 4-Hydroxy-1-(2-hydroxypropyl)-2,2,6,6-tetramethylpiperidin 4-(2,3-Epoxypropyloxy-1-(2-hydroxypropyl)-2,2,6,6-tetramethylpiperidin, Sdp. 118-120 °C/3,999 Pa

aus 4-Hydroxy-2,2,6,6-tetramethylpiperidin 4-(2,3-Epoxypropyloxy)-2,2,6,6-tetramethylpiperidin. Sdp. 54 °C/1,333 Pa

aus 4-Hydroxy-1-(2,3-epoxypropyl)-2,2,6,6-tetramethylpiperidin 4-(2,3-Epoxypropyloxy)-1-(2,3-epoxypropyl)-2,2,6,6-tetramethylpiperidin, Sdp. 120 °C/0,666 Pa

aus 4-Hydroxy-1-(2-hydroxyethyl)-2,2,6,6-tetramethylpiperidin 4-(2,3-Epoxypropyloxy)-1-(5,6-epoxy-3-oxahexyl)-2,2,6,6-tetramethylpiperidin, Sdp. 150-155 °C/0,666 Pa

aus 1-(2-hydroxyethyl)-2,2,6,6-tetramethylpiperidin 1-(5,6-Epoxy-3-oxa-hexyl)-2,2,6,6-tetramethylpiperidin, Sdp. 88-90 °C.

## Beispiel 5

Härtung eines Acryl-Melaminharz-Lackes

Es wird ein Klarlack der folgenden Rezeptur bereitet :

| | |
|---|---|
| 57,30 g | Hydroxylfunktionelles Acrylharz (Paraloid® AT 410, Rohm & Haas, USA) |
| 17,93 g | Hexamethoxymethylmelamin (Cyme 1® 301, Amer Cyanamid) |
| 1,83 g | Celluloseacetobutyrat (CAB 551.001, Eastman Chem. Co.) |
| 2,80 g | Verlaufshilfsmittel auf Basis eines Silikonharzes (Byketol® Spezial, Byk-Mallinchrodt, BRD) |
| 0,29 g | Verlaufshilfsmittel auf Basis eines Tensides (Modaflow®, Monsanto, USA) |
| 10,12 g | Butanol |
| 9,73 g | Butylacetat |
| 100,00 g | |

Dieser Lack hat einen Festkörpergehalt von 59,76 %. Proben dieses Lackes werden mit den in Tabelle 1 und 2 angegebenen Mengen von verschiedenen Härtungskatalysatoren vermischt und in einer Trockenfilmstärke von ca. 40 µm auf mit einem weissen Coil-Coat beschichtete Aluminiumbleche aufgebracht. Die Härtung geschieht 30 Minuten bei 100 °C. Zur Beurteilung des Härtungsgrades wird die

Pendelhärte nach König (DIN 53 157) bestimmt, und zwar 30 Minuten nach dem Einbrennen.

Zur Beurteilung der Verfärbung (Vergilbung) wird der Farbtonabstand ΔE gemäss DIN 6 174 mit einem Hunterlab-Photometer gemessen.

Zur Beurteilung der Lagerstabilität des Lackes wird seine Viskosität (mit einem ICI-Kegel-Platte Viskosimeter) während 7-tägiger Lagerung bei 70 °C gemessen.

Die Resultate sind in den Tabellen 1 und 2 aufgeführt.

Tabelle 1 — Härtung 30 Minuten bei 120 °C

| Härtungskatalysator (Gew.-% bezogen auf Festkörper) | Pendelhärte (Sek.) | Farbtonabstand ΔE |
|---|---|---|
| keiner | keine Härtung | |
| 1% p-Toluolsulfonsäure | 194 | 0,1 |
| 0,5% Verbindung Nr. 9 | 127 | 0,4 |
| 1% Verbindung Nr. 9' | 188 | 0,2 |
| 1% Verbindung Nr. 10 | 127 | 0,2 |
| 2% Verbindung Nr. 10 | 119 | 0,4 |
| 1% Verbindung Nr. 11 | 88 | 0,3 |
| 1% Verbindung Nr. 12 | 172 | 0,2 |

Tabelle 2 — Relativer Viskositätsanstieg Δη bei 7-tägiger Lagerung bei 60 °C.

| Härtungskatalysator | Δη (in %) |
|---|---|
| keiner | 16 |
| 1% p-Toluolsulfonsäure | geliert |
| 0,5% Verbindung Nr. 9 | 128 |
| 1% Verbindung Nr. 9 | 140 |
| 1% Verbindung Nr. 10 | 24 |
| 2% Verbindung Nr. 10 | 30 |
| 1% Verbindung Nr. 11 | 50 |
| 1% Verbindung Nr. 12 | 600 |

**Patentansprüche**

1. Verbindungen der Formeln I und II

$$B—(X—O—SO_2—A)_m \quad (I)$$

$$(B'—X—O—SO_2)_2—A' \quad (II),$$

worin m die Zahlen 1 bis 3 bedeutet,

X eine direkte Bindung oder eine Gruppe der Formel III

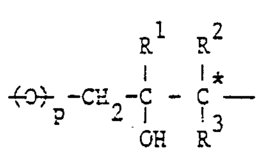

(III)

ist, die über C* an den Sauerstoff der Sulfogruppe gebunden ist, wobei, falls die Gruppe der Formel III an ein Stickstoffatom von B oder B' gebunden ist, p Null bedeutet und sonst p die Zahl 1 ist, und worin $R^1$, $R^2$ und $R^3$ unabhängig voneinander Wasserstoff oder Methyl bedeuten,

A unsubstituiertes oder durch Hydroxy substituiertes $C_1$-$C_{20}$ Alkyl, $C_1$-$C_4$ Alkoxy, $C_2$-$C_{12}$ Alkenyl, $C_7$-$C_{12}$ Aralkyl, $C_6$-$C_{10}$ Aryl, $C_7$-$C_{30}$ Alkaryl, $C_5$-$C_8$ Cycloalkyl, Trifluormethyl, Campheryl, eine Gruppe $-N(R^4)(R^5)$ oder $-C(CH_3)_2-CH_2-N(R^6)-C(O)-C(R^7)=CH_2$, worin $R^4$, $R^5$ und $R^6$ Wasserstoff oder $C_1$-$C_{12}$ Alkyl und $R^7$ Wasserstoff oder Methyl sind, eine Gruppe der Formel

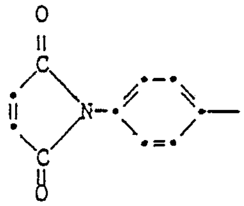

oder der Formel

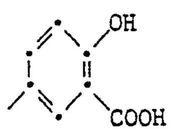

bedeuten,

A' $C_2$-$C_{20}$ Alkylen, $C_6$-$C_{12}$ Arylen oder $C_7$-$C_{28}$ Alkylarylen ist,

B bei m = 1 und B' eine Gruppe der Formeln IV bis X bedeuten,

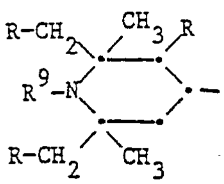

(IV)

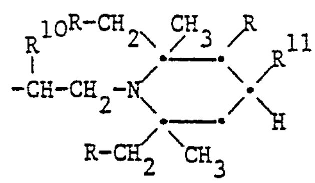

(V)

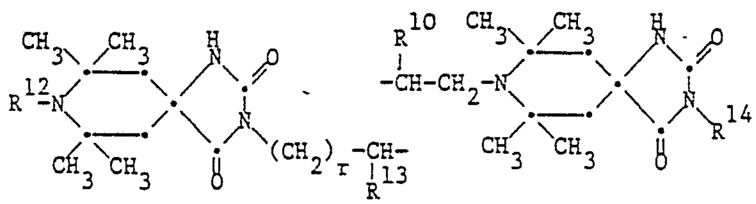

(VI) (VII)

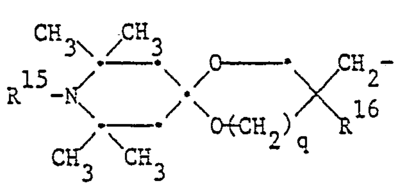

(VIII)

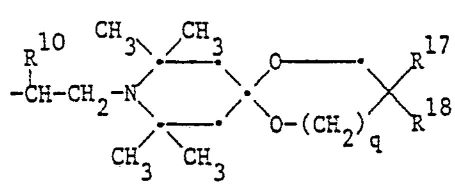

(IX)

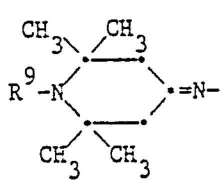

(X)

B bei m = 1 und B', falls X eine Gruppe der Formel III bedeutet, zusätzlich auch eine Gruppe der Formeln XI bis XVI sein kann,

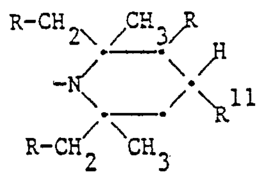

(XI)

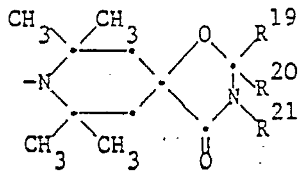

(XII)

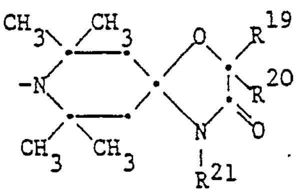

(XIII)

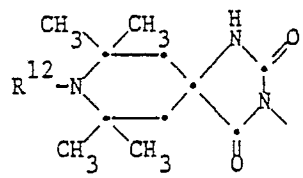

(XIV)

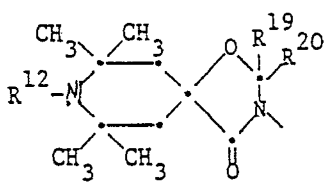

(XV)

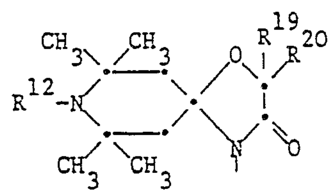

(XVI)

B bei m = 2 eine Gruppe der Formeln XVII bis XX bedeutet,

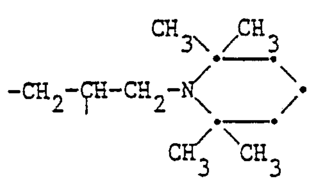

(XVII)

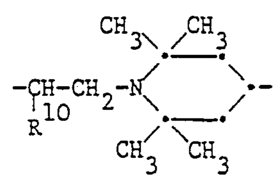

(XVIII)

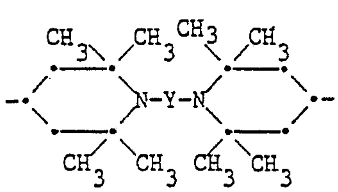

(XIX)

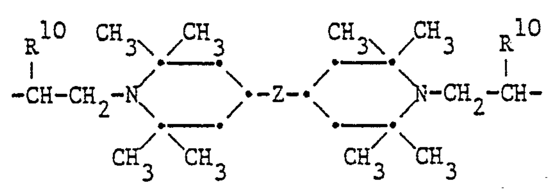

(XX)

B bei m = 2, falls X eine Gruppe der Formel III bedeutet, zusätzlich auch eine Gruppe der Formeln XXI bis XXV sein kann,

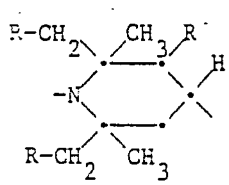

(XXI)

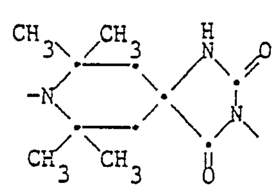

(XXII)

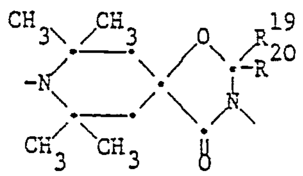

(XXIII)

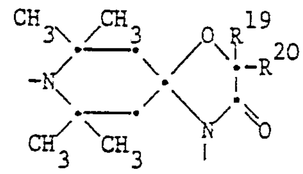

(XXIV)

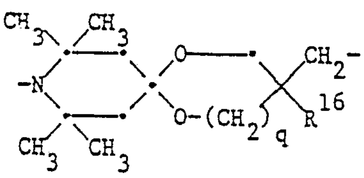

(XXV)

B bei m = 3, eine Gruppe der Formel XXVI

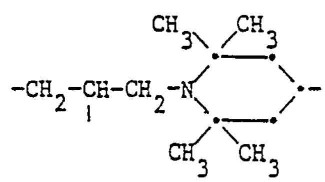

(XXVI)

bedeutet, wobei in den Formeln IV bis XXV

R Wasserstoff oder Methyl,

$R^9$ Wasserstoff, $C_1$-$C_{20}$ Alkyl, $C_3$-$C_5$ Alkenyl, $C_3$-$C_5$ Alkinyl, $C_7$-$C_{12}$ Aralkyl, $C_1$-$C_{18}$ Acyl, $C_3$-$C_{10}$ Alkoxycarbonylalkyl, $C_2$-$C_4$ Hydroxyalkyl, Cyanomethyl, Oxyl, eine Gruppe der Formel $-CH_2-CH(OH)-CH_2-OR^{27}$ oder eine Gruppe der Formel $-C(O)-N(R^{22})(R^{23})$,

$R^{10}$ Wasserstoff, $C_1$-$C_4$ Alkyl, $C_1$-$C_4$ Alkoxy, Phenyl, unsubstituiertes oder durch $C_1$-$C_4$ Alkyl im Phenylkern substituiertes Phenoxy, Phenoxymethyl,

$R^{11}$ Wasserstoff, Hydroxy, $C_1$-$C_{12}$ Alkoxy, $C_3$-$C_5$ Alkenyloxy, $C_1$-$C_{12}$ Acyloxy, $C_1$-$C_{12}$ Acylamino, $C_2$-$C_{14}$ Alkoxycarbonyl, eine Gruppe der Formel

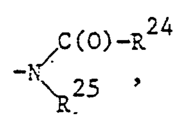 , $-C(O)-N(R^{24})(R^{25})$, $-OC(O)-N(R^{24})(R^{25})$,

oder $-CH_2-COOR^{24}$,

$R^{12}$ Wasserstoff, $C_1$-$C_{12}$ Alkyl, $C_3$-$C_5$ Alkenyl, $C_3$-$C_5$ Alkinyl, $C_7$-$C_{12}$ Aralkyl, $C_1$-$C_{18}$ Acyl, Cyanomethyl, $C_2$-$C_4$ Hydroxyalkyl,

$R^{13}$ Wasserstoff, $C_1$-$C_4$ Alkyl,

$R^{14}$ Wasserstoff, $C_1$-$C_{20}$ Alkyl, $C_3$-$C_5$ Alkenyl, $C_3$-$C_5$ Alkinyl, $C_7$-$C_{12}$ Aralkyl, $C_6$-$C_8$ Cycloalkyl,

$R^{15}$ Wasserstoff, $C_1$-$C_{12}$ Alkyl, $C_3$-$C_5$ Alkenyl, $C_7$-$C_{12}$ Aralkyl, $C_1$-$C_{18}$ Acyl, Cyanomethyl, $C_2$-$C_4$ Hydroxyalkyl,

$R^{16}$ Wasserstoff, Methyl, Ethyl,

$R^{17}$ und $R^{18}$ unabhängig voneinander Wasserstoff, $C_1$-$C_6$ Alkyl oder $R^{17}$ und $R^{18}$ zusammen eine der Gruppen der Formeln

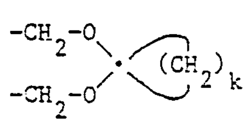 oder 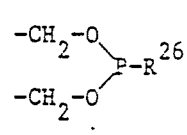 ,

$R^{19}$ und $R^{20}$ unabhängig voneinander Wasserstoff, $C_1$-$C_{12}$ Alkyl, unsubstituiertes oder durch $C_1$-$C_4$

Alkyl substituiertes Phenyl, $C_5$-$C_8$ Cycloalkyl, $C_7$-$C_{12}$ Aralklyl oder $R^{19}$ und $R^{20}$ zusammen $C_4$-$C_{11}$ Alkylen,

$R^{21}$ Wasserstoff, $C_1$-$C_{12}$ Alkyl, $C_3$-$C_5$ Alkenyl, $C_1$-$C_{18}$ Acyl, $C_7$-$C_{12}$ Aralkyl,

$R^{22}$ und $R^{23}$ unabhängig voneinander Wasserstoff, $C_1$-$C_{12}$ Alkyl, $C_3$-$C_5$ Alkenyl, Phenyl, Cyclohexyl,

$R^{24}$ $C_1$-$C_{12}$ Alkyl,

$R^{25}$ Wasserstoff oder $C_1$-$C_{12}$ Alkyl,

$R^{26}$ $C_1$-$C_4$ Alkyl, $C_1$-$C_{12}$ Alkoxy, Phenyl, Benzyl, Phenoxy,

$R^{27}$ $C_1$-$C_4$ Alkyl oder Phenyl,

r die Zahlen 1 bis 4,

q die Zahlen Null oder 1

k die Zahlen 4 bis 11,

Y Ethylen, 2-Butenylen-1,4, o-, p- oder m-Xylylen,

Z eine Gruppe der Formeln $-O-R^{28}-O-$, $-OC(O)-R^{29}-C(O)O-$, $N(R^{30})-C(O)-R^{31}-C(O)-N(R^{30})-$, $-N(R^{30})-C(O)-C(O)-N(R^{30})-$,

$-C(O)O-R^{31}-OC(O)-$, 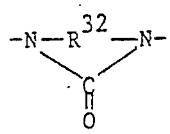

$R^{28}$ $C_2$-$C_{20}$ Alkylen, o-, m- oder p-Xylylen, 1,2-, 1,3- oder 1,4-Dimethylencyclohexylen,

$R^{29}$ $C_2$-$C_{20}$ Alkylen, o-, m- oder p-Phenylen, $C_8$-$C_{12}$ Arylendialkylen,

$R^{30}$ Wasserstoff, $C_1$-$C_{12}$ Alkyl, $C_7$-$C_{12}$ Aralkyl, $C_5$-$C_8$ Cycloalkyl, unsubstituiertes oder durch $C_1$-$C_4$ Alkyl substituiertes Phenyl,

$R^{31}$ $C_2$-$C_{20}$ Alkylen, unsubstituiertes oder durch $C_1$-$C_4$ Alkyl substituiertes o-, m- oder p-Phenylen, 1,2-, 1,3- oder 1,4-Cyclohexylen,

$R^{32}$ Ethylen oder Propylen bedeuten

sowie deren Säureadditionssalze.

2. Verbindungen gemäss Anspruch 1 der Formel I, worin m die Zahlen 1 oder 2 bedeutet,

X eine direkte Bindung oder eine Gruppe der Formel III, worin $R^1$, $R^2$ und $R^3$ Wasserstoff sind, bedeutet,

A $C_1$-$C_{18}$ Alkyl, Methoxy, Vinyl, Amino, Phenyl, Naphthyl, 1 oder 2 mal durch $C_1$-$C_{12}$ Alkyl substituiertes Phenyl oder Naphthyl, 2,4,6-Trimethylphenyl, Campher-10-yl, eine Gruppe der Formel $-C(CH_3)_2-CH_2-NH-C(O)-CH=CH_2$ oder eine Gruppe der Formel

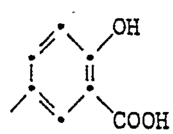

ist,

B bei m = 1 eine Gruppe der Formeln IV und V ist, bei m = 1, falls X eine Gruppe der Formel III bedeutet, zusätzlich auch eine Gruppe der Formeln XI und XIV ist,

bei m = 2 eine Gruppe der Formeln XVIII und XX ist und

bei m = 2, falls X eine Gruppe der Formel III bedeutet, zusätzlich auch eine Gruppe der Formel XXI bedeutet,

wobei in den Formeln IV, V, XI, XIV, XVIII, XX und XXI

R Wasserstoff,

$R^9$ Wasserstoff, $C_1$-$C_6$ Alkyl, Allyl, Benzyl, Acetyl, Acryloyl, 2-Hydroxyethyl, Cyanomethyl oder Oxyl,

$R^{10}$ Wasserstoff, Methyl, Phenyl, Phenoxymethyl,

$R^{11}$ Wasserstoff, $C_1$-$C_4$ Alkoxy, Allyloxy, $C_1$-$C_{12}$ Acyloxy, $C_1$-$C_{12}$ Acylamino, $C_2$-$C_6$ Alkoxycarbonyl oder eine Gruppe der Formel

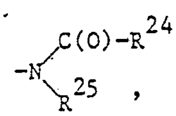 ,

$R^{12}$ Wasserstoff oder $C_1$-$C_4$ Alkyl,

$R^{13}$ Wasserstoff,

$R^{14}$ Wasserstoff, $C_1$-$C_{12}$ Alkyl, Allyl oder Benzyl,

$R^{15}$ Wasserstoff, Methyl oder Benzyl,

$R^{16}$ Wasserstoff, Methyl oder Ethyl,

$R^{17}$ und $R^{18}$ $C_1$-$C_4$ Alkyl,
$R^{19}$ und $R^{20}$ Wasserstoff, $C_1$-$C_6$ Alkyl oder zusammen $C_4$-$C_{11}$ Alkylen,
$R^{24}$ $C_1$-$C_{12}$ Alkyl
$R^{25}$ Wasserstoff oder $C_1$-$C_{12}$ Alkyl sind,
Z eine Gruppe der Formeln $—OC(O)—R^{29}—C(O)O—$ oder $—N(R^{30})—C(O)—R^{31}—C(O)—N(R^{30})—$ bedeutet,
worin
$R^{29}$ $C_2$-$C_{10}$ Alkylen, o-, m-, oder p-Phenylen,
$R^{30}$ Wasserstoff, $C_1$-$C_{12}$ Alkyl oder Benzyl und
$R^{31}$ $C_2$-$C_{10}$ Alkylen, o-, m- oder p-Phenylen sind,
sowie deren Säureadditionssalze.

3. Verbindungen gemäss Anspruch 1 der Formel I, worin m die Zahlen 1 oder 2 bedeutet, X eine direkte Bindung oder eine Gruppe der Formel III, worin $R^1$, $R^2$ und $R^3$ Wasserstoff sind, bedeutet,
A Methyl, Ethyl, $C_{14}$-$C_{17}$ Alkyl, Methoxy, Vinyl, Amino, Phenyl, p-Methylphenyl, p-Isopropylphenyl, p-Dodecylphenyl, Naphthyl, Dinonylnaphthyl, Campher-10-yl, eine Gruppe der Formel $—C(CH_3)_2—CH_2—NH—C(O)—CH=CH_2$ oder eine Gruppe der Formel

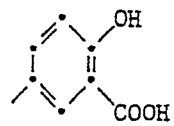

ist,
B bei m = 1 eine Gruppe der Formeln IV und V ist,
bei m = 1, falls X eine Gruppe der Formel III bedeutet, zusätzlich auch eine Gruppe der Formel XI ist,
bei m = 2 eine Gruppe der Formel XVIII ist und
bei m = 2, falls X eine Gruppe der Formel III bedeutet, zusätzlich auch eine Gruppe der Formel XXI bedeutet,
wobei in den Formeln IV, V, XI, XVIII und XXI
R Wasserstoff,
$R^9$ Wasserstoff, $C_1$-$C_6$ Alkyl, Allyl, Benzyl, Oxyl, Acetyl, Acryloyl, β-Hydroxyethyl oder Cyanomethyl,
$R^{10}$ Wasserstoff, Methyl oder Phenyl und
$R^{11}$ Wasserstoff oder $C_1$-$C_4$ Alkoxy sind,
sowie deren Säureadditionssalze.

4. Verbindungen gemäss Anspruch 3 der Formel I, worin m die Zahl 1 bedeutet, B eine Gruppe der Formel IV ist, X eine direkte Bindung oder eine Gruppe der Formel $—O—CH_2—CH(OH)—CH_2—$ ist und A die in Anspruch 3 gegebene Bedeutung hat.

5. Verwendung der Verbindungen der Formel I gemäss Anspruch 1 zur Härtung von säurehärtbaren Harzen.

6. Stoffzusammensetzungen enthaltend ein säurehärtbares Harz und eine Verbindung der Formel I gemäss Anspruch 1.

7. Stoffzusammensetzung gemäss Anspruch 6, dadurch gekennzeichnet, dass das säurehärtbare Harz eine Acrylatkomponente enthält und mit vollverätherten Melaminharzen gehärtet wird.

**Claims**

1. A compound of the formulae I and II

$$B—(X—O—SO_2—A)_m \quad (I)$$

$$(B'—X—O—SO_2)_2—A' \quad (II)$$

in which m is a number from 1 to 3,
X is a direct bond or a group of the formula III

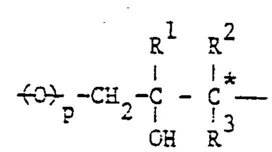

(III)

which is attached via C* to the oxygen of the sulfo group, p being zero if the group of the formula III is attached to a nitrogen atom of B or B', and p otherwise being the number 1, and each of $R^1$, $R^2$ and $R^3$ independently of one another being hydrogen or methyl,

A is $C_1$-$C_{20}$-alkyl, $C_1$-$C_4$-alkoxy, $C_2$-$C_{12}$-alkenyl, $C_7$-$C_{12}$-aralkyl, $C_6$-$C_{10}$-aryl, $C_7$-$C_{30}$-alkaryl, $C_5$-$C_8$-cycloalkyl, trifluoromethyl or camphoryl, each of which is unsubstituted or substituted by hydroxy, or A is a group $-N(R^4)(R^5)$ or $-C(CH_3)_2-CH_2-N(R^6)-C(O)-C(R^7)=CH_2$, in which $R^4$, $R^5$ and $R^6$ are hydrogen or $C_1$-$C_{12}$-alkyl and $R^7$ is hydrogen or methyl, or A is a group of the formula

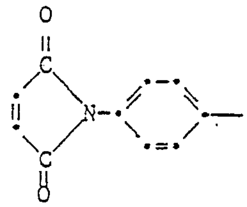

or of the formula

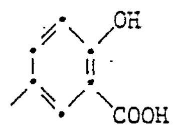

A' is $C_2$-$C_{20}$-alkylene, $C_6$-$C_{12}$-arylene or $C_7$-$C_{28}$-alkylarylene, and B, if m is 1, and B' are a group of the formulae IV to X

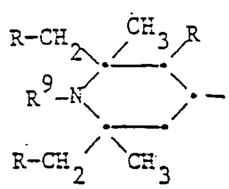

(IV)

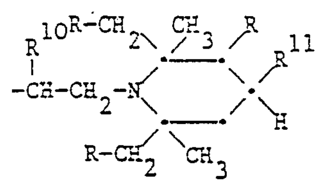

(V)

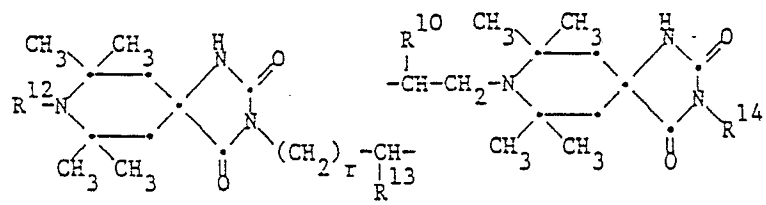

(VI)

(VII)

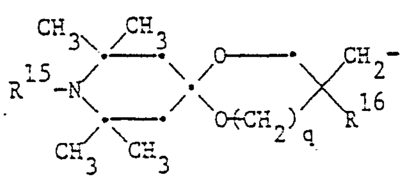

(VIII)

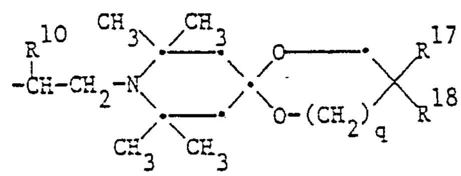

(IX)

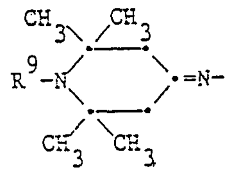

(X)

in addition, B, if m is 1, and B', if X is a group of the formula III, can also be a group of the formulae XI to XVI

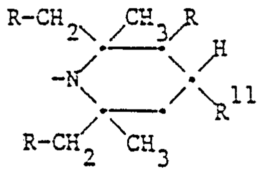

(XI)

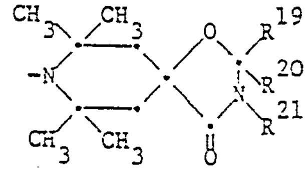

(XII)

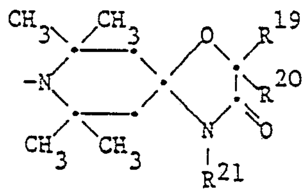

(XIII)

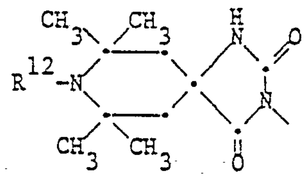

(XIV)

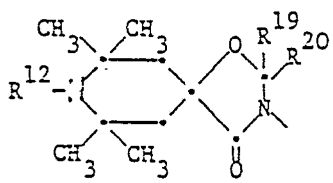

(XV)

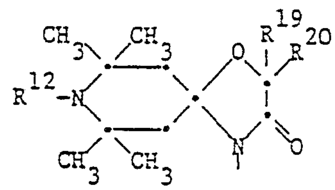

(XVI)

if m is 2, B is a group of the formulae XVII to XX

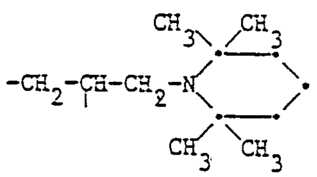

(XVII)

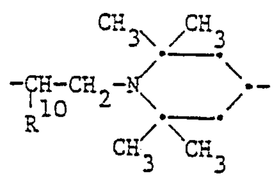

(XVIII)

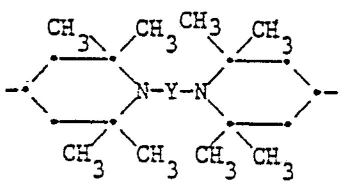

(XIX)

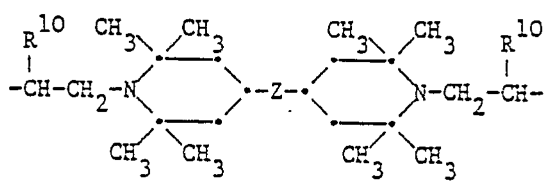

(XX)

in addition, if m is 2 and if X is a group of the formula III, B can also be a group of the formulae XXI to XXV

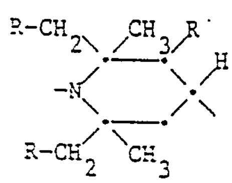

(XXI)

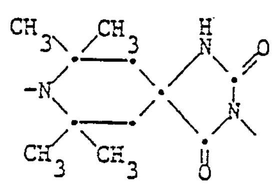

(XXII)

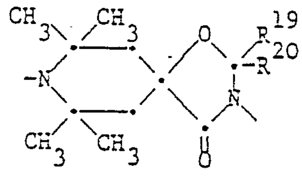

(XXIII)

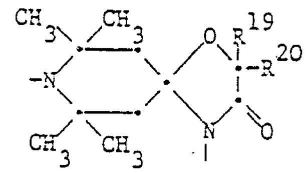

(XXIV)

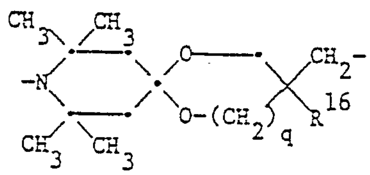

(XXV)

and, if m is 3, B is a group of the formula XXVI

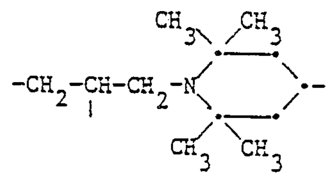

(XXVI)

and, in the formulae IV to XXV,

R is hydrogen or methyl,

$R^9$ is hydrogen, $C_1$-$C_{20}$-alkyl, $C_3$-$C_5$-alkenyl, $C_3$-$C_5$-alkynyl, $C_7$-$C_{12}$-aralkyl, $C_1$-$C_{18}$-acyl, $C_3$-$C_{10}$-alkoxycarbonylalkyl, $C_2$-$C_4$-hydroxyalkyl, cyanomethyl, oxyl, a group of the formula $—CH_2—CH(OH)—CH_2—OR^{27}$ or a group of the formula $—C(O)—N(R^{22})\ (R^{23})$,

$R^{10}$ is hydrogen, $C_1$-$C_4$-alkyl, $C_1$-$C_4$-alkoxy, phenyl, phenoxy which is unsubstituted or substituted in the phenyl nucleus by $C_1$-$C_4$-alkyl, or is phenoxymethyl,

$R^{11}$ is hydrogen, hydroxy, $C_1$-$C_{12}$-alkoxy, $C_3$-$C_5$-alkenyloxy, $C_1$-$C_{12}$-acyloxy, $C_1$-$C_{12}$-acylamino, $C_2$-$C_{14}$-alkoxycarbonyl or a group of the formula

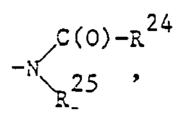 , $-C(O)-N(R^{24})(R^{25})$, $-OC(O)-N(R^{24})(R^{25})$,

or $—CH_2—COOR^{24}$,

$R^{12}$ is hydrogen, $C_1$-$C_{12}$-alkyl, $C_3$-$C_5$-alkenyl, $C_3$-$C_5$-alkynyl, $C_7$-$C_{12}$-aralkyl, $C_1$-$C_{18}$-acyl, cyanomethyl or $C_2$-$C_4$-hydroxyalkyl,

$R^{13}$ is hydrogen or $C_1$-$C_4$-alkyl,

$R^{14}$ is hydrogen, $C_1$-$C_{20}$-alkyl, $C_3$-$C_5$-alkenyl, $C_3$-$C_5$-alkynyl, $C_7$-$C_{12}$-aralkyl or $C_6$-$C_8$-cycloalkyl,

$R^{15}$ is hydrogen, $C_1$-$C_{12}$-alkyl, $C_3$-$C_5$-alkenyl, $C_7$-$C_{12}$-aralkyl, $C_1$-$C_{18}$-acyl, cyanomethyl or $C_2$-$C_4$-hydroxyalkyl,

$R^{16}$ is hydrogen, methyl or ethyl,

each of $R^{17}$ and $R^{18}$ independently of the other is hydrogen or $C_1$-$C_6$-alkyl or $R^{17}$ and $R^{18}$ together are one of the groups of the formulae

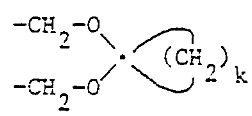 or 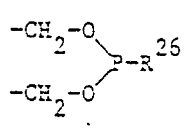 ,

each of $R^{19}$ and $R^{20}$ independently of the other is hydrogen, $C_1$-$C_{12}$-alkyl, phenyl which is

unsubstituted or substituted by $C_1$-$C_4$-alkyl, or is $C_5$-$C_8$-cycloalkyl or $C_7$-$C_{12}$-aralkyl or $R^{19}$ and $R^{20}$ together are $C_4$-$C_{11}$-alkylene,

$R^{21}$ is hydrogen, $C_1$-$C_{12}$-alkyl, $C_3$-$C_5$-alkenyl, $C_1$-$C_{18}$-acyl or $C_7$-$C_{12}$-aralkyl,

each of $R^{22}$ and $R^{23}$ independently of the other is hydrogen, $C_1$-$C_{12}$-alkyl, $C_3$-$C_5$-alkenyl, phenyl or cyclohexyl,

$R^{24}$ is $C_1$-$C_{12}$-alkyl,

$R^{25}$ is hydrogen or $C_1$-$C_{12}$-alkyl,

$R^{26}$ is $C_1$-$C_4$-alkyl, $C_1$-$C_{12}$-alkoxy, phenyl, benzyl or phenoxy,

$R^{27}$ is $C_1$-$C_4$-alkyl or phenyl,

r is a number from 1 to 4,

q is the number 0 or 1,

k is a number from 4 to 11,

Y is ethylene, 1,4-but-2-enylene or o-, p- or m-xylylene,

Z is a group of the formula $—O—R^{28}—O—$, $—OC(O)—R^{29}—C(O)O—$, $—N(R^{30})—C(O)—R^{31}—C(O)—N(R^{30})—$, $—N(R^{30})—C(O)—C(O)—N(R^{30})$,

$-C(O)O-R^{31}-OC(O)-$, 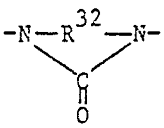

$R^{28}$ is $C_2$-$C_{20}$-alkylene, o-, m- or p-xylylene or 1,2-, 1,3- or 1,4-dimethylenecyclohexylene,

$R^{29}$ is $C_2$-$C_{20}$-alkylene, o-, m- or p-phenylene or $C_8$-$C_{12}$-arylenedialkylene,

$R^{30}$ is hydrogen, $C_1$-$C_{12}$-alkyl, $C_7$-$C_{12}$-aralkyl, $C_5$-$C_8$-cycloalkyl, or phenyl which is unsubstituted or substituted by $C_1$-$C_4$-alkyl,

$R^{31}$ is $C_2$-$C_{20}$-alkylene or o-, m- or p-phenylene or 1,2-, 1,3- or 1,4-cyclohexylene, each of which is unsubstituted or substituted by $C_1$-$C_4$-alkyl, and

$R^{32}$ is ethylene or propylene,

or an acid addition salt thereof.

2. A compound according to claim 1 of the formula I, in which m is the number 1 or 2,

X is a direct bond or a group of the formula III in which $R^1$, $R^2$ and $R^3$ are hydrogen,

A is $C_1$-$C_{18}$-alkyl, methoxy, vinyl, amino, phenyl or naphthyl, or phenyl or naphthyl, each of which is monosubstituted or disubstituted by $C_1$-$C_{12}$-alkyl, or A is 2,4,6-trimethylphenyl, camphor-10-yl, a group of the formula $—C(CH_3)_2—CH_2—NH—C(O)—CH{=}CH_2$ or a group of the formula

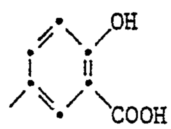

and B, if m is 1, is a group of the formulae IV and V, and, if m is 1 and if X is a group of the formula III, B is, in addition, also a group of the formulae XI and XIV, and, if m

m is 2, B is a group of the formulae XVIII and XX, and, if

m is 2 and if X is a group of the formula III, B is, in addition, also a group of the formula XXI, and, in the formulae IV, V, XI, XIV, XVIII, XX and XXI,

R is hydrogen,

$R^9$ is hydrogen, $C_1$-$C_6$-alkyl, allyl, benzyl, acetyl, acryloyl, 2-hydroxyethyl, cyanomethyl or oxyl,

$R^{10}$ is hydrogen, methyl, phenyl or phenoxymethyl,

$R^{11}$ is hydrogen, $C_1$-$C_4$-alkoxy, allyloxy, $C_1$-$C_{12}$-acyloxy, $C_1$-$C_{12}$-acylamino, $C_2$-$C_6$-alkoxycarbonyl or a group of the formula

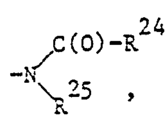 ,

$R^{12}$ is hydrogen or $C_1$-$C_4$-alkyl,

$R^{13}$ is hydrogen,

$R^{14}$ is hydrogen, $C_1$-$C_{12}$-alkyl, allyl or benzyl,

$R^{15}$ is hydrogen, methyl or benzyl,

$R^{16}$ is hydrogen, methyl or ethyl,

$R^{17}$ and $R^{18}$ are $C_1$-$C_4$-alkyl,

$R^{19}$ and $R^{20}$ are hydrogen or $C_1$-$C_6$-alkyl, or, together, are $C_4$-$C_{11}$-alkylene,

$R^{24}$ is $C_1$-$C_{12}$-alkyl,

$R^{25}$ is hydrogen or $C_1$-$C_{12}$-alkyl and

Z is a group of the formulae —OC(O)—$R^{29}$—C(O)O— or —N($R^{30}$)—C(O)—$R^{31}$—C(O)—N($R^{30}$)—, in which

$R^{29}$ is $C_2$-$C_{10}$-alkylene or o-, m- or p-phenylene,

$R^{30}$ is hydrogen, $C_1$-$C_{12}$-alkyl or benzyl and

$R^{31}$ is $C_2$-$C_{10}$-alkylene or o-, m- or p-phenylene,

or an acid addition salt thereof.

3. A compound according to claim 1 of the formula I, in which m is the number 1 or 2,

X is a direct bond or a group of the formula III in which $R^1$, $R^2$ and $R^3$ are hydrogen,

A is methyl, ethyl, $C_{14}$-$C_{17}$-alkyl, methoxy, vinyl, amino, phenyl, p-methylphenyl, p-isopropylphenyl, p-dodecylphenyl, naphthyl, dinonylnaphthyl, camphor-10-yl, a group of the formula —$C(CH_3)_2$—$CH_2$—NH—C(O)—CH=$CH_2$ or a group of the formula

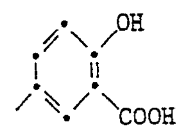

and B, if m is 1, is a group of the formulae IV and V, and,

if m is 1 and if X is a group of the formula III, B is, in addition, also a group of the formula XI and,

if m is 2, B is a group of the formula XVIII and,

if m is 2 and if X is a group of the formula III, B is, in addition, also a group of the formula XXI, and, in the formulae IV, V, XI, XVIII and XXI,

R is hydrogen,

$R^9$ is hydrogen, $C_1$-$C_6$-alkyl, allyl, benzyl, oxyl, acetyl, acryloyl, β-hydroxyethyl or cyanomethyl,

$R^{10}$ is hydrogen, methyl or phenyl and

$R^{11}$ is hydrogen or $C_1$-$C_4$-alkoxy,

or an acid addition salt thereof.

4. A compound according to claim 3 of the formula I, in which m is the number 1, B is a group of the formula IV, X is a direct bond or a group of the formula —O—$CH_2$—CH(OH)—$CH_2$— and A is as defined in claim 3.

5. Use of a compound of the formula I according to claim 1 for curing acid-curable resins.

6. A composition containing an acid-curable resin and a compound of the formula I according to claim 1.

7. A composition according to claim 6, in which the acid-curable resin contains an acrylate component and is cured with completely etherified melamine resins.

**Revendications**

1. Composés répondant à l'une des formules I et II :

$$B—(X—O—SO_2—A)_m \qquad (I)$$

$$(B'—X—O—SO_2)_2—A' \qquad (II)$$

dans lesquelles :

m représente un nombre de 1 à 3,

X représente une liaison directe ou un radical de formule III :

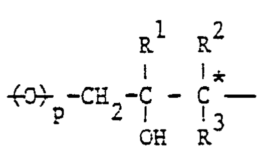

(III)

qui est relié par C* à l'oxygène du radical sulfo et dans lequel p est égal à 0 lorsque le radical (III) est relié à un atome d'azote de B ou de B' et est égal à 1 dans les autres cas, les symboles $R^1$, $R^2$ et $R^3$ représente chacun, indépendamment l'un de l'autre, l'hydrogène ou un radical méthyle,

A représente un alkyle en $C_1$-$C_{20}$ non substitué ou porteur d'un hydroxy, un alcoxy en $C_1$-$C_4$, un alcényle en $C_2$-$C_{12}$, un aralkyle en $C_7$-$C_{12}$, un aryle en $C_6$-$C_{10}$, un alkylaryle en $C_7$-$C_{30}$, un cycloalkyle en $C_5$-$C_8$, un trifluorométhyle, un camphéryle, un radical de formule —N($R^4$) ($R^5$) ou de formule —$C(CH_3)_2$—$CH_2$—N($R^6$)—C(O)—C($R^7$)=$CH_2$, dans lesquelles $R^4$, $R^5$ et $R^6$ représentent chacun l'hydrogène ou un alkyle en $C_1$-$C_{12}$ et $R^7$ représente l'hydrogène ou un méthyle, ou A représente un radical de formule :

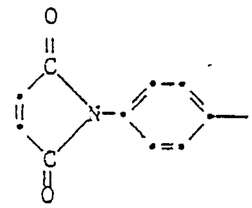

ou de formule :

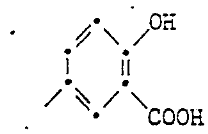

A' représente un alkylène en $C_2$-$C_{20}$, un arylène en $C_6$-$C_{12}$ ou un alkylarylène en $C_7$-$C_{28}$.

B, lorsque m est égal à 1, et B' représentent chacun un radical répondant à l'une des formules IV à X :

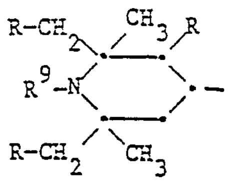

(IV)

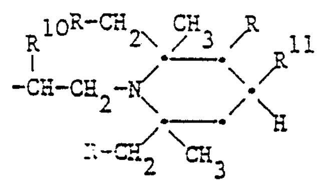

(V)

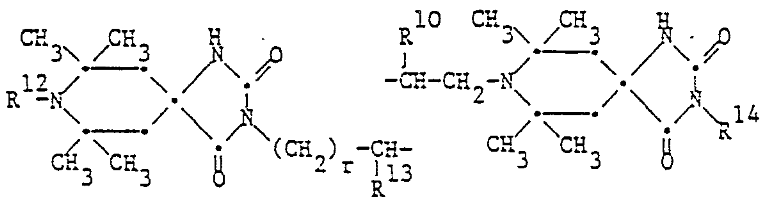

(VI) (VII)

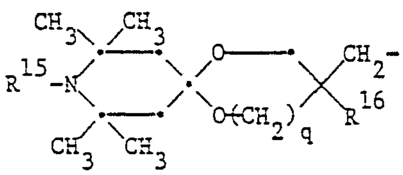

(VIII)

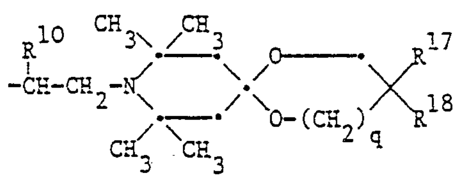

(IX)

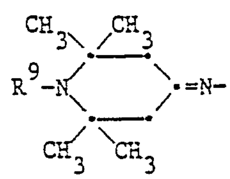

(X)

B, lorsque m est égal à 1, et B' peuvent en outre, dans le cas où X désigne un radical de formule III, représenter chacun un radical répondant à l'une des formules XI à XVI :

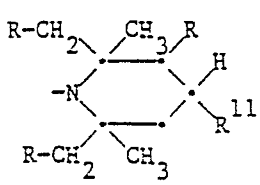

(XI)

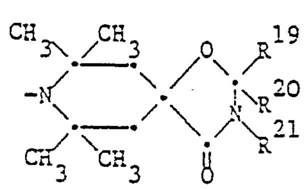

(XII)

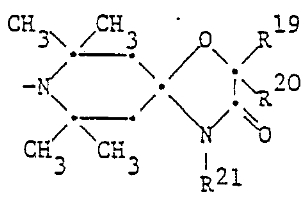

(XIII)

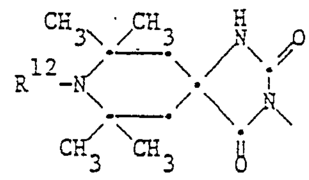

(XIV)

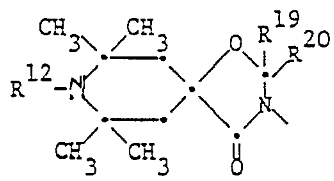

(XV)

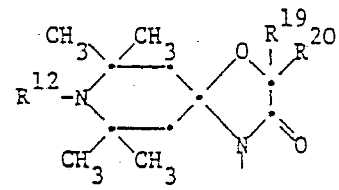

(XVI)

B représente, lorsque m est égal à 2, un radical répondant à l'une des formules XVII à XX :

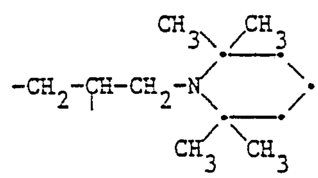

(XVII)

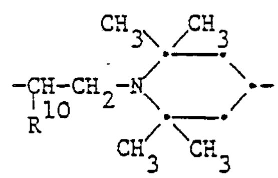

(XVIII)

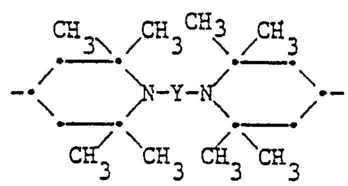

(XIX)

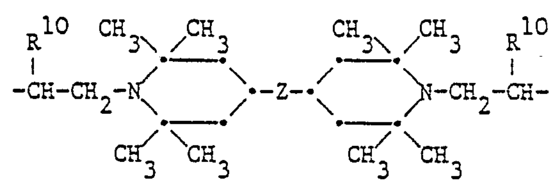

(XX)

B peut en outre représenter, lorsque m est égal à 2 et que X représente un radical de formule III, un radical répondant à l'une des formules XXI à XXV :

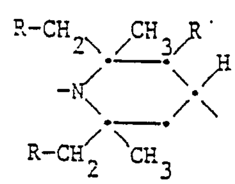

(XXI)

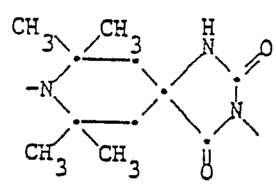

(XXII)

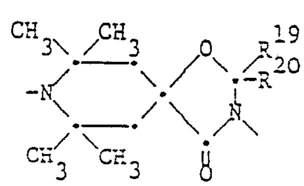

(XXIII)

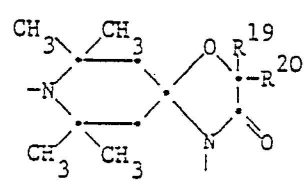

(XXIV)

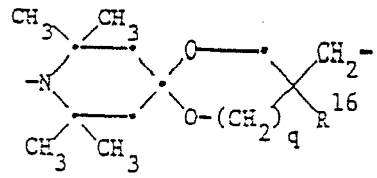

(XXV)

B représente, lorsque m est égal à 3, un radical de formule XXVI :

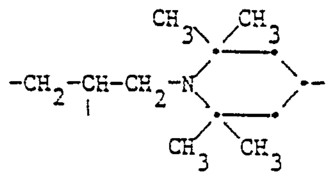

(XXVI)

les divers symboles présents dans les formules IV à XXV ayant les significations suivantes :

R représente l'hydrogène ou un méthyle,

$R^9$ représente l'hydrogène, un alkyle en $C_1$-$C_{20}$, un alcényle en $C_3$-$C_5$, un alcynyle en $C_3$-$C_5$, un aralkyle en $C_7$-$C_{12}$, un acyle en $C_1$-$C_{18}$, un alcoxycarbonylalkyle en $C_3$-$C_{10}$, un hydroxyalkyle en $C_2$-$C_4$, un cyanométhyle, un oxyle, un radical de formule $—CH_2—CH(OH)—CH_2OR^{27}$ ou un radical de formule $—C(O)—N(R^{22})(R^{23})$,

$R^{10}$ représente l'hydrogène, un alkyle en $C_1$-$C_4$, un alcoxy en $C_1$-$C_4$, un phényle, un phenoxy non substitué ou porteur d'un alkyle en $C_1$-$C_4$ sur son noyau phényle, ou un phénoxyméthyle,

$R^{11}$ représente l'hydrogène, un hydroxy, un alcoxy en $C_1$-$C_{12}$, un alcényloxy en $C_3$-$C_5$, un acyloxy en $C_1$-$C_{12}$, un acylamino en $C_1$-$C_{12}$, un alcoxycarbonyle en $C_2$-$C_{14}$ ou un radical répondant à l'une des formules :

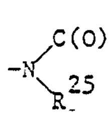 , $-C(O)-N(R^{24})(R^{25})$, $-OC(O)-N(R^{24})(R^{25})$,

et

$—CH_2—COOR^{24}$

$R^{12}$ représente l'hydrogène, un alkyle en $C_1$-$C_{12}$, un alcényle en $C_3$-$C_5$, un alcynyle en $C_3$-$C_5$, un aralkyle en $C_7$-$C_{12}$, un acyle en $C_1$-$C_{18}$, un cyanométhyle ou un hydroxyalkyle en $C_2$-$C_4$,

$R^{13}$ représente l'hydrogène ou un alkyle en $C_1$-$C_4$,

$R^{14}$ représente l'hydrogène, un alkyle en $C_1$-$C_{20}$, un alcényle en $C_3$-$C_5$, un alcynyle en $C_3$-$C_5$, un aralkyle en $C_7$-$C_{12}$ ou un cycloalkyle en $C_6$-$C_8$,

$R^{15}$ représente l'hydrogène, un alkyle en $C_1$-$C_{12}$, un alcényle en $C_3$-$C_5$, un aralkyle en $C_7$-$C_{12}$, un acyle en $C_1$-$C_{18}$, un cyanométhyle ou un hydroxyalkyle en $C_2$-$C_4$,

$R^{16}$ représente l'hydrogène, un méthyle ou un éthyle,

$R^{17}$ et $R^{18}$ représentent chacun, indépendamment l'un de l'autre, l'hydrogène ou un alkyle en $C_1$-$C_6$, ou encore $R^{17}$ et $R^{18}$ forment ensemble un radical répondant à l'une des formules :

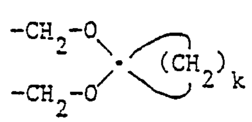 et 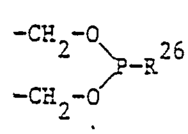 ,

$R^{19}$ et $R^{20}$ représentent chacun, indépendamment l'un de l'autre, l'hydrogène, un alkyle en $C_1$-$C_{12}$, un phényle non substitué ou porteur d'un alkyle en $C_1$-$C_4$, un cycloalkyle en $C_5$-$C_8$ ou un aralkyle en $C_7$-$C_{12}$, ou encore $R^{19}$ et $R^{20}$ forment ensemble un alkylène en $C_4$-$C_{11}$,

$R^{21}$ représente l'hydrogène, un alkyle en $C_1$-$C_{12}$, un alcényle en $C_3$-$C_5$, un acyle en $C_1$-$C_{18}$ ou un aralkyle en $C_7$-$C_{12}$,

$R^{22}$ et $R^{23}$ représentent chacun, indépendamment l'un de l'autre, l'hydrogène, un alkyle en $C_1$-$C_{12}$, un alcényle en $C_3$-$C_5$, un phényle ou un cyclohexyle,

$R^{24}$ représente un alkyle en $C_1$-$C_{12}$,

$R^{25}$ représente l'hydrogène ou un alkyle en $C_1$-$C_{12}$,

$R^{26}$ représente un alkyle en $C_1$-$C_4$, un alcoxy en $C_1$-$C_{12}$, un phényle, un benzyle ou un phénoxy.

$R^{27}$ représente un alkyle en $C_1$-$C_4$ ou un phényle,

r représente un nombre de 1 à 4,

q représente le nombre 0 ou 1,

k représente un nombre de 4 à 11,

Y représente un radical éthylène, butène-2 ylène-1,4 ou o-, m- ou p-xylylène,

Z représente un radical —O—$R^{28}$—O—, —OC(O)—$R^{29}$—C(O)O—, —N($R^{30}$)—C(O)—$R^{31}$—C(O)—N($R^{30}$)—, —N($R^{30}$)—C(O)—C—(O)—N($R^{30}$)—,

-C(O)O-$R^{31}$-OC(O)-, 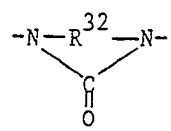

$R^{28}$ représente un alkylène en $C_2$-$C_{20}$, un radical o-, m- ou p-xylylène ou un radical cyclohexylène-diméthylène-1,2, -1,3 ou -1,4,

$R^{29}$ représente un alkylène en $C_2$-$C_{20}$, un radical o-, m- ou p-phénylène ou un radical arylène-dialkylène en $C_8$-$C_{12}$,

$R^{30}$ représente l'hydrogène, un alkyle en $C_1$-$C_{12}$, un aralkyle en $C_7$-$C_{12}$, un cycloalkyle en $C_5$-$C_8$ ou un radical phényle non substitué ou porteur d'un alkyle en $C_1$-$C_4$,

$R^{31}$ représente un alkylène en $C_2$-$C_{20}$, un radical o-, m- ou p-phénylène non substitué ou porteur d'un alkyle en $C_1$-$C_4$, ou un radical cyclohexylène-1,2, -1,3 ou -1,4, et

$R^{32}$ représente un radical éthylène ou propylène,

ainsi que les sels d'addition qu'ils forment avec des acides.

2. Composés de formule I selon la revendication 1 dans lesquels :

m est égal à 1 ou à 2,

X représente une liaison directe ou un radical de formule III dans lequel $R^1$, $R^2$ et $R^3$ représentent chacun l'hydrogène,

A représente un alkyle en $C_1$-$C_{18}$, un méthoxy, un vinyle, un amino, un phényle, un naphtyle, un radical phényle ou naphtyle porteur d'un ou deux alkyles en $C_1$-$C_{12}$, un triméthyl-2,4,6 phényle, un camphéryle-10, un radical —$C(CH_3)_2$—$CH_2$—NH—C(O)—CH=$CH_2$ ou un radical de formule :

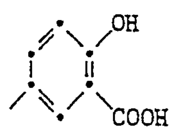

B représente :

dans le cas où m est égal à 1, un radical de formule IV ou V,

dans le cas où m est égal à 1 et X est un radical de formule III, il peut en outre représenter un radical de formule XI ou XIV,

dans le cas où m est égal à 2, un radical de formule XVIII ou XX et

dans le cas où m est égal à 2 et qu'en outre X représente un radical de formule III, il peut également représenter un radical de formule XXI,

les différents symboles présents dans les formules IV, V, XI, (XIV), XVIII, XX et XXI ayant les significations suivantes :

R représente l'hydrogène,

$R^9$ représente l'hydrogène, un alkyle en $C_1$-$C_6$, un allyle, un benzyle, un acétyle, un acryloyle, un hydroxy-2 éthyle, un cyanométhyle ou un oxyle,

$R^{10}$ représente l'hydrogène, un méthyle, un phényle ou un phénoxyméthyle,

$R^{11}$ représente l'hydrogène, un alcoxy en $C_1$-$C_4$, un allyloxy, un acyloxy en $C_1$-$C_{12}$, un acylamino en $C_1$-$C_{12}$, un alcoxycarbonyle en $C_2$-$C_6$ ou un radical de formule :

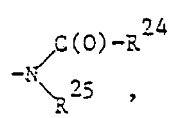 ,

$R^{12}$ représente l'hydrogène ou un alkyle en $C_1$-$C_4$,

$R^{13}$ représente l'hydrogène,

$R^{14}$ représente l'hydrogène, un alkyle en $C_1$-$C_{12}$, un allyle ou un benzyle,

$R^{15}$ représente l'hydrogène, un méthyle ou un benzyle,

$R^{16}$ représente l'hydrogène, un méthyle ou un éthyle,

$R^{17}$ et $R^{18}$ représentent chacun un alkyle en $C_1$-$C_4$,

$R^{19}$ et $R^{20}$ représentent chacun l'hydrogène ou un alkyle en $C_1$-$C_6$ ou forment ensemble un alkylène en $C_4$-$C_{11}$,

$R^{24}$ représente un alkyle en $C_1$-$C_{12}$,

$R^{25}$ représente l'hydrogène ou un alkyle en $C_1$-$C_{12}$ et

Z représente un radical de formule —OC(O)—$R^{29}$—C(O)O— ou de formule —N($R^{30}$)—C(O)—$R^{31}$—C(O)—N($R^{30}$)—, formules dans lesquelles :

$R^{29}$ représente un alkylène en $C_2$-$C_{10}$ ou un radical o-, m- ou p-phénylène,

$R^{30}$ représente l'hydrogène, un alkyle en $C_1$-$C_{12}$ ou un benzyle et

$R^{31}$ représente un alkylène en $C_2$-$C_{10}$ ou un radical o-, m- ou p-phénylène,

ainsi que les sels d'addition qu'ils forment avec des acides.

3. Composés de formule I selon la revendication 1 dans lesquels :

m est égal à 1 ou à 2,

X représente une liaison directe ou un radical de formule III dans lequel $R^1$, $R^2$ et $R^3$ représentent chacun l'hydrogène,

A représente un méthyle, un éthyle, un alkyle en $C_{14}$-$C_{17}$, un méthoxy, un vinyle, un amino, un phényle, un p-méthylphényle, un p-isopropylphényle, un p-dodécylphényle, un naphtyle, un dinonylnaphtyle, un camphéryle-10, un radical de formule —$C(CH_3)_2$—$CH_2$—NH—C(O)—CH=$CH_2$ ou un radical de formule :

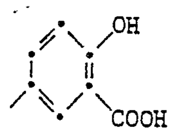

B représente :

dans le cas où m est égal à 1, un radical de formule IV ou V,

dans le cas où m est égal à 1 et où X est un radical de formule III, il peut en outre représenter un radical de formule XI,

dans le cas où m est égal à 2, un radical de formule XVIII, et

dans le cas où m est égal à 2 et où X est un radical de formule III, il peut en outre représenter un radical de formule XXI,

les symboles présents dans les formules IV, V, XI, XVIII et XXI ayant les significations suivantes :

R représente l'hydrogène,

$R^9$ représente l'hydrogène, un alkyle en $C_1$-$C_6$, un allyle, un benzyle, un oxyle, un acétyle, un acryloyle, un hydroxy-2 éthyle ou un cyanométhyle,

$R^{10}$ représente l'hydrogène, un méthyle ou un phényle, et

$R^{11}$ représente l'hydrogène ou un alcoxy en $C_1$-$C_4$,

ainsi que les sels d'addition qu'ils forment avec des acides.

4. Composés de formule I selon la revendication 3 dans lesquels m représente le nombre 1, B représente un radical de formule IV, X représente une liaison directe ou un radical —O—$CH_2$—CH(OH)—$CH_2$— et A a la signification qui lui a été donnée à la revendication 3.

5. Application des composés de formule I selon la revendication 1 au durcissement de résines durcissables par des acides.

6. Composition contenant une résine durcissable par des acides et un composé de formule I selon la revendication 1.

7. Composition selon la revendication 6 caractérisée en ce que la résine durcissable par des acides contient une composante acrylique et est durcie par des résines de mélamine totalement éthérifiées.